# EUROPEAN PATENT APPLICATION

(11) **EP 4 682 893 A1**
(43) Date of publication of application: **21.01.2026**
(21) Application number: 24770860.5
(22) Date of filing: 11.03.2024
(51) Int. Cl.: G16C 60/00, C22C 21/06, G06N 3/044, G06N 3/0455, G16C 20/30, G16C 20/70

(54) **PREDICTION RESULT ANALYSIS DEVICE, ANALYSIS METHOD, AND ANALYSIS PROGRAM**

(30) Priority: 13.03.2023 JP 2023038608
(71) Applicant: Resonac Corporation, Tokyo 105-7325 (JP)
(72) Inventor: OKANO, Yu, Tokyo 105-7325 (JP); KANESHITA, Takeshi, Tokyo 105-7325 (JP)
(74) Representative: Strehl & Partner mbB
(86) International application number: PCT/JP2024/009428
(87) International publication number: WO 2024/190755

(57) **Abstract**

One object is to easily understand the degree of impact on a phase ratio caused by a modification of a material composition. A prediction result analysis device includes a storage unit configured to store a trained model that is trained using training data in which a material composition of a material to be trained, and a phase ratio of the material to be trained at each temperature within a specific temperature range, are associated with each other, an intermediate composition generation unit configured to generate a material composition of a plurality of intermediate materials, by dividing between the material composition of the material to be predicted and a material composition of a baseline material, a prediction unit configured to calculate a partial derivative by partially differentiating a value predicted for a phase specified in advance at each temperature, when predicting a phase ratio of the plurality of intermediate materials at each temperature within the specific temperature range, by inputting the material composition of the plurality of intermediate materials to the trained prediction model, an integral gradient calculation unit configured to calculate an integral gradient of each temperature and each component, by integrating the calculated partial derivative for each component included in the material composition of the plurality of intermediate materials at each temperature, and a display unit configured to display a heat map in which each component is arranged on a first axis and each temperature is arranged on a second axis, and each area specified based on each component and each temperature is displayed in a display mode corresponding to the integral gradient.

## Description

### TECHNICAL FIELD

The present disclosure relates to prediction result analysis devices, analysis methods, and analysis programs.

### BACKGROUND ART

When designing materials, it is important to calculate a phase ratio in a thermodynamic equilibrium state over a specific temperature range, and a surrogate model for predicting the relevant phase ratio based on material composition information is being developed. For example, a surrogate model for predicting the relevant phase ratio based on a content of an additive element of an alloy is being developed.

The phase ratio within the specific temperature range is correlated to material properties. For this reason, when a designer designs the alloy using the model described above, it is important from a viewpoint of efficiently designing the material that a designer understands in advance which phase of which temperature range can be affected by increasing or decreasing the content of which additive element, in order to obtain a phase ratio close to a desired phase ratio.

### PRIOR ART DOCUMENTS

### PATENT DOCUMENTS

Patent Document 1: International Publication Pamphlet No. WO 2020/090617

### DISCLOSURE OF THE INVENTION

### PROBLEM TO BE SOLVED BY THE PRESENT INVENTION

In contrast, the designer may perform an operation of predicting the phase ratio within a specific temperature range while successively increasing or decreasing the content of all the additive elements, and comparing the predicted phase ratio with the phase ratio before the modification, for example. This is because, a degree of impact of the content of each additive element on the phase ratio can be understood in advance by performing such an operation.

However, according to such an operation, if the alloy or the like includes a large number of additive elements, a work load on the designer increases. In addition, it is difficult for the designer to understand the degree of impact of the content of each additive element on the phase ratio, by merely comparing the phase ratios.

One object of the present disclosure is to facilitate understanding of the degree of impact on the phase ratio caused by a modification of a material composition.

### MEANS OF SOLVING THE PROBLEM

According to a first aspect of the present disclosure, a prediction result analysis device includes:
a storage unit configured to store a trained model that is trained using training data in which a material composition of a material to be trained, and a phase ratio of the material to be trained at each temperature within a specific temperature range, are associated with each other, the trained model being configured to predict a phase ratio at an (i+1)-th temperature using phase ratios predicted by the trained model for temperatures to an i-th temperature (i is an integer greater than or equal to 1) within the specific temperature range;
an intermediate composition generation unit configured to generate a material composition of a plurality of intermediate materials, by dividing between the material composition of the material to be predicted and a material composition of a baseline material;
a prediction unit configured to calculate a partial derivative by partially differentiating a value predicted for a phase specified in advance at each temperature, when predicting a phase ratio of the plurality of intermediate materials at each temperature within the specific temperature range, by inputting the material composition of the plurality of intermediate materials to the trained prediction model;
an integral gradient calculation unit configured to calculate an integral gradient of each temperature and each component, by integrating the calculated partial derivative for each component included in the material composition of the plurality of intermediate materials at each temperature; and
a display unit configured to display a heat map in which each component is arranged on a first axis and each temperature is arranged on a second axis, and each area specified based on each component and each temperature is displayed in a display mode corresponding to the integral gradient.

According to a second aspect of the present disclosure, in the first aspect, the integral gradient calculation unit calculates the integral gradient of each temperature and each component, by integrating the calculated partial derivative of each temperature for each component included in the material composition of the plurality of intermediate materials using a composition weight calculated in advance for each intermediate material of the plurality of intermediate materials, and averaging the integrated partial derivative.

According to a third aspect of the present disclosure, in the second aspect, the integral gradient calculation unit calculates the composition weight, based on a difference between each material composition of the plurality of intermediate materials, and the material composition of the material to be predicted.

According to a fourth aspect of the present disclosure, in the third aspect, the integral gradient calculation unit calculates the composition weight, based on a difference between each material composition of the plurality of intermediate materials, and the material composition of the material to be predicted.

According to a fifth aspect of the present disclosure, in any one of the first through fourth aspects:
the intermediate composition generation unit generates the material composition of the plurality of intermediate materials, for each material composition of a plurality of baseline materials,
the integral gradient calculation unit calculates the integral gradient of each temperature and each component, for each material composition of the plurality of baseline materials, and
the display unit displays the heat map in a display mode according to an averaged integral gradient acquired by averaging a plurality of integral gradients of each temperature and each component.

According to a sixth aspect of the present disclosure, in any one of the first through fifth aspects, an architecture capable of processing time series data which are data at specific time intervals is applied to the trained model, and the trained model predicts a phase ratio for each specific temperature interval based on the material composition of the material to be predicted.

According to a seventh aspect of the present disclosure, in the sixth aspect, the trained model is any one of an RNN, a bidirectional RNN, a Seq2Seq, a Seq2Seq with Attention mechanism, a GRU, an LSTM, and a Transformer.

According to an eighth aspect of the present disclosure, in the seventh aspect, the trained model includes:
an encoder unit configured to output a feature, in response to an input of the material composition of the material to be predicted, and
a decoder unit configured to predict the phase ratio at the (i+1)-th temperature, in response to an input of the output feature and predicted phase ratios for the temperatures to the i-th temperature.

According to a ninth aspect of the present disclosure, in any one of the first through eighth aspects, the phase ratio is a phase ratio in a thermodynamic equilibrium state.

According to a tenth aspect of the present disclosure, a prediction result analysis method to be implemented in a computer for a trained model that is trained using training data in which a material composition of a material to be trained, and a phase ratio of the material to be trained at each temperature within a specific temperature range, are associated with each other, the trained model being configured to predict a phase ratio at an (i+1)-th temperature using phase ratios predicted by the trained model for temperatures to an i-th temperature (i is an integer greater than or equal to 1) within the specific temperature range, the prediction result analysis method including the steps of:
generating, by the computer, a material composition of a plurality of intermediate materials, by dividing between the material composition of the material to be predicted and a material composition of a baseline material;
calculating, by the computer, a partial derivative by partially differentiating a value predicted for a phase specified in advance at each temperature, when predicting a phase ratio of the plurality of intermediate materials at each temperature within the specific temperature range, by inputting the material composition of the plurality of intermediate materials to the trained prediction model;
calculating, by the computer, an integral gradient of each temperature and each component, by integrating the calculated partial derivative for each component included in the material composition of the plurality of intermediate materials at each temperature; and
displaying, by the computer, a heat map in which each component is arranged on a first axis and each temperature is arranged on a second axis, and each area specified based on each component and each temperature is displayed in a display mode corresponding to the integral gradient.

According to an eleventh aspect of the present disclosure, a prediction program for causing a computer to implement a trained model that is trained using training data in which a material composition of a material to be trained, and a phase ratio of the material to be trained at each temperature within a specific temperature range, are associated with each other, the trained model being configured to predict a phase ratio at an (i+1)-th temperature using phase ratios predicted by the trained model for temperatures to an i-th temperature (i is an integer greater than or equal to 1) within the specific temperature range, the prediction program causing the computer to perform the steps of:
generating a material composition of a plurality of intermediate materials, by dividing between the material composition of the material to be predicted and a material composition of a baseline material;
calculating a partial derivative by partially differentiating a value predicted for a phase specified in advance at each temperature, when predicting a phase ratio of the plurality of intermediate materials at each temperature within the specific temperature range, by inputting the material composition of the plurality of intermediate materials to the trained prediction model;
calculating an integral gradient of each temperature and each component, by integrating the calculated partial derivative for each component included in the material composition of the plurality of intermediate materials at each temperature; and
displaying a heat map in which each component is arranged on a first axis and each temperature is arranged on a second axis, and each area specified based on each component and each temperature is displayed in a display mode corresponding to the integral gradient.

### EFFECTS OF THE PRESENT INVENTION

According to the present disclosure, it is possible to facilitate understanding of the degree of impact on the phase ratio caused by a modification of a material composition.

### BRIEF DESCRIPTION OF THE DRAWINGS

[FIG. 1] FIG. 1 is a diagram illustrating an example of a system configuration of a prediction system and examples of functional configurations of a training device, a prediction device, and a prediction result analysis device.
[FIG. 2] FIG. 2 is a diagram illustrating examples of hardware configurations of the training device, the prediction device, and the prediction result analysis device.
[FIG. 3] FIG. 3 is a diagram illustrating a configuration of training data and specific examples of input data and ground truth.
[FIG. 4] FIG. 4 is a diagram illustrating an example of a functional configuration of a training data generation unit.
[FIG. 5] FIG. 5 is a diagram illustrating an example of a functional configuration of a training unit.
[FIG. 6] FIG. 6 is a first diagram illustrating an example of an operation of the training unit.
[FIG. 7] FIG. 7 is a second diagram illustrating the example of the operation of the training unit.
[FIG. 8] FIG. 8 is a diagram illustrating an example of a method of calculating a loss by a loss function calculation unit.
[FIG. 9] FIG. 9 is a flow chart illustrating a flow of a training process.
[FIG. 10] FIG. 10 is a first diagram illustrating an example of a functional configuration of a prediction unit.
[FIG. 11] FIG. 11 is a first diagram illustrating an example of an operation of the prediction unit.
[FIG. 12] FIG. 12 is a flow chart illustrating a flow of a prediction process.
[FIG. 13] FIG. 13 is a diagram for explaining a prediction accuracy of a phase ratio.
[FIG. 14] FIG. 14 is a first diagram illustrating a specific example of a process of the prediction result analysis device.
[FIG. 15] FIG. 15 is a second diagram illustrating an example of the functional configuration of the prediction unit.
[FIG. 16A]FIG. 16A is a second diagram illustrating an example of the operation of the prediction unit.
[FIG. 16B]FIG. 16B is a third diagram illustrating an example of the operation of the prediction unit.
[FIG. 16C]FIG. 16C is a fourth diagram illustrating an example of the operation of the prediction unit.
[FIG. 17] FIG. 17 is a second diagram illustrating a specific example of the process of the prediction result analysis device.
[FIG. 18] FIG. 18 is a flow chart illustrating a flow of a prediction result analysis process.
[FIG. 19] FIG. 19 is a flow chart illustrating a flow of a baseline composition information input process.
[FIG. 20] FIG. 20 is a flow chart illustrating a flow of a integral gradient calculation process.
[FIG. 21] FIG. 21 is a diagram illustrating examples of a material composition and a predicted phase ratio of a material to be predicted used in the prediction result analysis process.
[FIG. 22] FIG. 22 is a diagram illustrating an example of a heat map displayed in a display mode according to the integral gradient.

### MODE OF CARRYING OUT THE INVENTION

Hereinafter, each embodiment will be described with reference to the accompanying drawings. In the present specification and drawings, constituent elements having substantially the same functional configuration are designated by the same reference numerals, and a redundant description thereof will be omitted.

### [First Embodiment]

### <System Configuration of Prediction System, and Functional Configurations of Training Device, Prediction Device, and Prediction Result Analysis Device>

First, a system configuration of a prediction system including a prediction result analysis device according to a first embodiment, and functional configurations of a training device, a prediction device, and the prediction result analysis device included in the relevant prediction system will be described. In the present embodiment, the prediction system predicts a phase ratio at each temperature within a specific temperature range, based on material composition information. The phase ratio refers to a phase ratio in a thermodynamic equilibrium state, and the phase ratio in the thermodynamic equilibrium state will hereinafter simply be referred to as the "phase ratio".

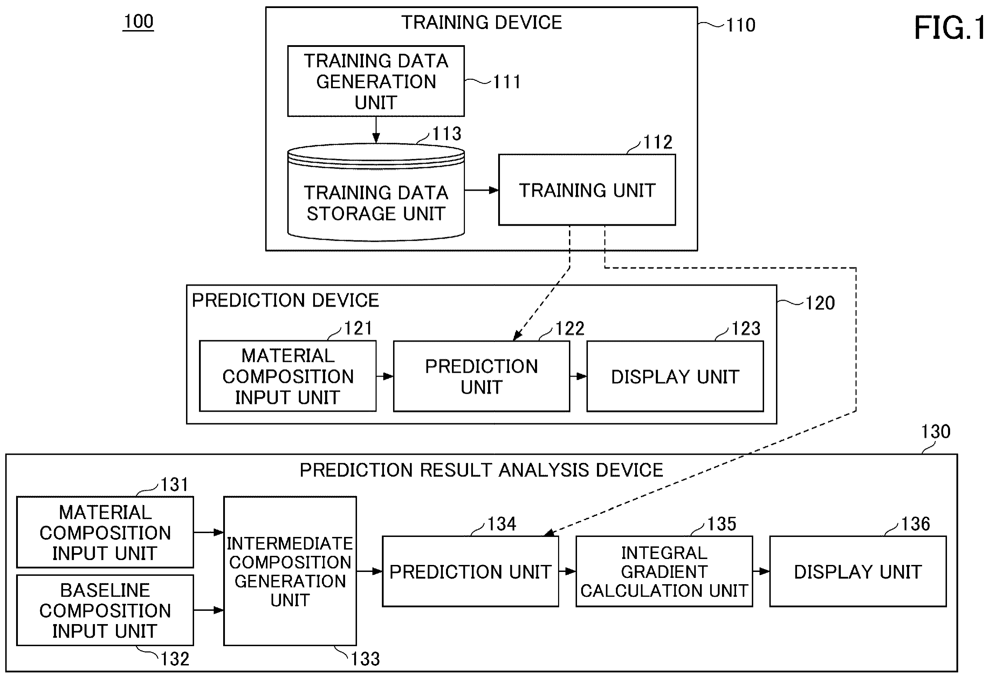

FIG. 1 is a diagram illustrating an example of the system configuration of the prediction system and examples of functional configurations of the training device, the prediction device, and the prediction result analysis device. As illustrated in FIG. 1, a prediction system 100 includes a training device 110, a prediction device 120, and a prediction result analysis device 130.

A training program is installed in the training device 110, and the training device 110 functions as a training data generation unit 111, and a training unit 112 by executing the relevant training program.

The training data generation unit 111 generates training data for training a prediction model, and stores the generated training data in the training data storage unit 113. The prediction model in this case is a surrogate model replacing the conventional model with machine learning. In the present embodiment, the training data storage unit 113 stores, as the training data for training the prediction model:
- a plurality of material composition information of different combinations of contents and components; and
- a phase ratio of materials having the respective material composition information at each temperature within a specific temperature range,
in association with each other.

The training unit 112 reads the training data from a training data storage unit 113, and thereafter trains the prediction model using the read training data. The training unit 112 trains the prediction model so that output data, output by inputting the material composition information stored in the training data to the prediction model, approaches the phase ratio at each temperature within the specific temperature range stored in association with the training data. Accordingly, the training unit 112 generates a trained prediction model. The training unit 112 stores the generated trained prediction model in a storage unit of a prediction unit 122 of the prediction device 120 and in a storage unit of a prediction unit 134 of the prediction result analysis device 130.

In the present embodiment, the training unit 112 implements, as the prediction model, an architecture capable of processing time series data which are data at specific time intervals, which is generally an architecture used for natural language processing or the like, such as:
- Seq2Seq with Attention mechanism; or
- Transformer.
Thus, according to the relevant prediction model, when the material composition information stored in the training data is input thereto, it is possible to successively output the output data corresponding to the phase ratio at each temperature within the specific temperature range.

The expression "successively output the output data corresponding to the phase ratio at each temperature" means that the prediction model outputs the output data corresponding to the phase ratio at an (i+1)-th temperature, using ground truth for each of the temperatures to the i-th temperature, for example. In this case, i is an integer greater than or equal to 1.

Accordingly, the training unit 112 has a configuration of successively outputting the output data corresponding to the phase ratio at each temperature. In other words, the training unit 112 has a configuration for processing the output data corresponding to the phase ratio at each temperature as time series data. Thus, the training unit 112 can perform a training which reflects the output data corresponding to the phase ratio in an adjacent temperature region.

A prediction program is installed in the prediction device 120, and the prediction device 120 functions as a material composition input unit 121, the prediction unit 122, and a display unit 123 by executing the relevant prediction program.

The material composition input unit 121 receives an input of the material composition information of a material to be predicted, and notifies the prediction unit 122 of the material composition information.

The prediction unit 122 stores the trained prediction model trained by the training unit 112 in the storage unit. The prediction unit 122 reads the trained prediction model from the storage unit, and thereafter inputs the material composition information notified from the material composition input unit 121 to the relevant trained prediction model, thereby successively predicting the phase ratio at each temperature within the specific temperature range. That is, the prediction unit 122 successively predicts the phase ratio within the specific temperature range for each of the specific temperature intervals.

The expression "successively predicting the phase ratio for each of the specific temperature intervals" means that the trained prediction model predicts the phase ratio at the (i+1)-th temperature, using the phase ratio predicted by the relevant trained prediction model for each of the temperatures to the i-th temperature (i is an integer greater than or equal to 1), for example.

As described above, in the present embodiment, the prediction unit 122 has a configuration for successively predicting the phase ratio at each temperature. That is, the prediction unit 122 has a configuration for processing the phase ratio at each temperature as the time series data by a recurrent neural network. Thus, the prediction unit 122 can perform a prediction which reflects prediction data of the phase ratio in the adjacent temperature region. As a result, compared to a case where a multilayer neural network is implemented, that is, a case where the prediction result of the phase ratio in the adjacent temperature region is not reflected), for example, it is possible to reduce a decrease in a prediction accuracy of the phase ratio. That is, according to the prediction unit 122, it is possible to improve the prediction accuracy when predicting the phase ratio over the specific temperature range based on the material composition.

The display unit 123 displays the phase ratio at each temperature within the specific temperature range predicted by the prediction unit 122. The display unit 123 displays the phase ratio at each temperature within the specific temperature range predicted by the prediction unit 122, in a different color for each phase. However, the phase ratio at each temperature within the specific temperature range predicted by the prediction unit 122 may be displayed by changing a line type, such as a solid line, a dotted line, a one-dot chain line, or the like for each phase.

An analysis program is installed in the prediction result analysis device 130. The prediction result analysis device 130 functions as a material composition input unit 131, a baseline composition input unit 132, an intermediate composition generation unit 133, the prediction unit 134, an integral gradient calculation unit 135, and a display unit 136 by executing the relevant program.

The material composition input unit 131 receives an input of material composition information of the material to be predicted, and notifies the intermediate composition generation unit 133 of the material composition information. The material composition information of the material to be predicted, which is input to the material composition input unit 131, is the same as the material composition information of the material to be predicted, which is input to the material composition input unit 121 of the prediction device 120.

The baseline composition input unit 132 receives an input of baseline composition information, which is material composition information of a material serving as a baseline, and notifies the intermediate composition generation unit 133 of the baseline composition information. The baseline composition information is material composition information used for calculating a gradient, when visualizing a basis of the prediction using an integrated gradients method.

For example, representative material composition information of the material to be predicted, material composition information including an upper limit value or a lower limit value of the composition of the material to be predicted, or the like is preferably used as the baseline composition information. In the case of 6000-series aluminum alloys, JIS includes A6061, A6063, or the like, and the upper limit and lower limit of the composition of the additive elements are defined for each standard. In this case, it is preferable that the material composition information including the upper limit value or the lower limit value of the additive element in the standard to which the material to be predicted belongs, is used as the baseline composition information.

In addition, when focusing on a specific precipitation phase, material composition information in which the specific precipitation phase is known to occur may be used as the baseline composition information.

By using a plurality of baseline composition information, the degree of impact on the phase ratio can be appropriately evaluated even for the phase ratio at which a specific additive element saturates.

The intermediate composition generation unit 133 generates intermediate composition information, which is material composition information of an intermediate material, based on the material composition information of the material to be predicted notified from the material composition input unit 131 and the baseline composition information notified from the baseline composition input unit 132. Specifically, the intermediate composition generation unit 133 generates the intermediate composition information by dividing between the material composition information of the material to be predicted and the baseline composition information by a number of steps = M, for example. Thus, the intermediate composition generation unit 133 can generate M intermediate composition information that gradually approach the baseline composition information, from the material composition information of the material to be predicted.

The intermediate composition generation unit 133 successively inputs the generated material composition information of the plurality of intermediate materials, that is, the M intermediate composition information, to the prediction unit 134.

The prediction unit 134 stores the trained prediction model, trained by the training unit 112, in the storage unit. The prediction unit 134 reads the trained prediction model from the storage unit, and thereafter inputs the M intermediate composition information notified from the intermediate composition generation unit 133 to the trained prediction model. Thus, the prediction unit 134 successively predicts the phase ratio at each temperature within the specific temperature range. That is, the prediction unit 122 successively predicts the phase ratio within the specific temperature range for each of the specific temperature intervals with respect to the M intermediate composition information.

When successively predicting the phase ratio within the specific temperature range for each of the specific temperature intervals, the prediction unit 134 partially differentiates the predicted value of the phase specified in advance at each temperature, using each of the M intermediate composition information. Thus, the prediction unit 134 can calculate a "partial derivative" indicating a varying extent of the phase specified in advance at each temperature, by varying each of the M intermediate composition information by a minute amount.

The integral gradient calculation unit 135 integrates the partial derivative of each of the M intermediate composition information for each temperature and each component, to calculate the integral gradient for each temperature and each component.

The display unit 136 applies a color scheme to each of areas specified based on each component and each temperature in a display mode according to the integral gradient, in a heat map in which each component is arranged on the abscissa and each temperature is arranged on the ordinate. The abscissa is an example of a first axis, and the ordinate is an example of a second axis. The display unit 136 displays the heat map with the color scheme in the display mode according to the integral gradient.

Accordingly, a designer of the material design can easily understand, based on the heat map displayed by the prediction result analysis device 130, how the material composition should be modified with respect to the phase ratio displayed by the prediction device 120, in order to obtain a phase ratio close to a desired phase ratio.

That is, according to the prediction result analysis device 130, when predicting the phase ratio based on the material composition information, the designer of the material design can easily understand the degree of impact of the modification of the material composition on the phase ratio, based on the heat map.

### <Hardware Configurations of Training Device, Prediction Device, and Prediction Result Analysis Device>

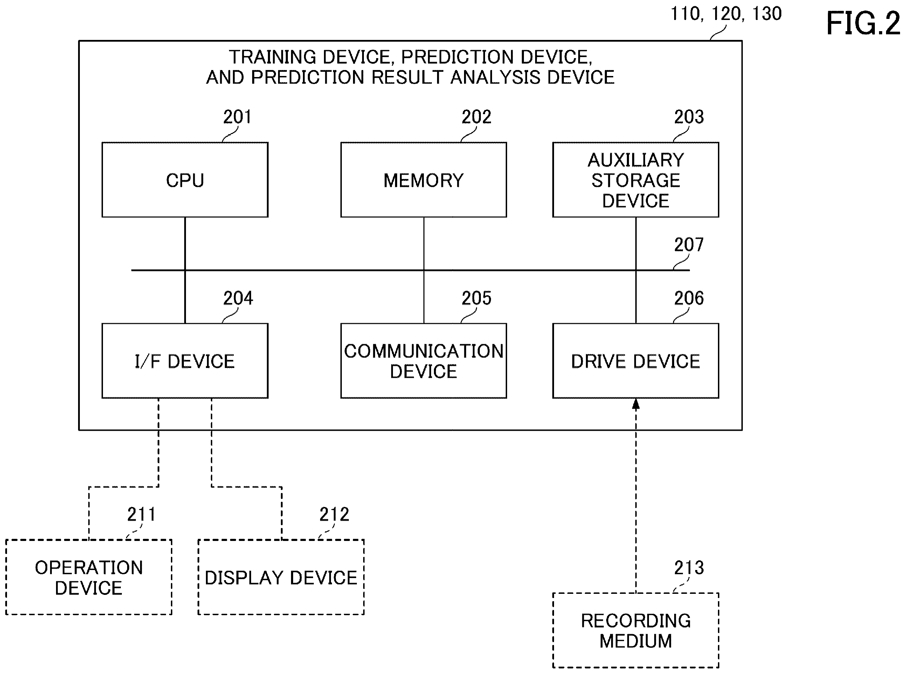

Next, hardware configurations of the training device 110, the prediction device 120, and the prediction result analysis device 130 will be described. Because the training device 110, the prediction device 120, and the prediction result analysis device 130 have the same hardware configuration, and the hardware configurations of the training device 110, the prediction device 120, and the prediction result analysis device 130 will be collectively described with reference to FIG. 2.

FIG. 2 is a diagram illustrating examples of hardware configurations of the training device, the prediction device, and the prediction result analysis device. As illustrated in FIG. 2, the training device 110, the prediction device 120, and the prediction result analysis device 130 include a processor 201, a memory 202, an auxiliary storage device 203, an interface device 204, a communication device 205, and a drive device 206. The hardware of the training device 110, the hardware of the prediction device 120, and the hardware of the prediction result analysis device 130 are connected to each other via a bus 207.

The processor 201 includes various computing devices, such as a central processing unit (CPU), a graphics processing unit (GPU), or the like. The processor 201 reads various programs into the memory 202 and executes the programs. The various programs include a training program, a prediction program, an analysis program, or the like, for example.

The memory 202 includes a main storage device, such as a read only memory (ROM), a random access memory (RAM), or the like. The processor 201 and the memory 202 form a so-called computer. The various functions described above are implemented by the computer when the processor 201 executes the various programs read into the memory 202.

The auxiliary storage device 203 stores various programs, and various data used when the various programs are executed by the processor 201. For example, the training data storage unit 113 is implemented by the auxiliary storage device 203. Alternatively, the storage unit that stores the trained prediction model is implemented by the auxiliary storage device 203.

The interface device 204 is a connection device that connects to the operation device 211 and the display device 212, which are examples of user interface devices. The communication device 205 is a communication device for communicating with an external apparatus (not illustrated) via a network.

The drive device 206 is a device to which a recording medium 213 is loaded. The recording medium 213 includes a medium for optically, electrically, or magnetically recording information, such as a CD-ROM, a flexible disk, a magneto-optical disk, or the like. The recording medium 213 may include a semiconductor memory or the like for electrically recording information, such as a ROM, a flash memory, or the like.

The various programs installed in the auxiliary storage device 203 are installed by loading the recording medium 213 that is distributed into the drive device 206, and reading the various programs recorded in the recording medium 213 by the drive device 206, for example. Alternatively, the various programs installed in the auxiliary storage device 203 may be installed by being downloaded from the network via the communication device 205.

### <Configuration of Training Data, Input Data, and Ground Truth>

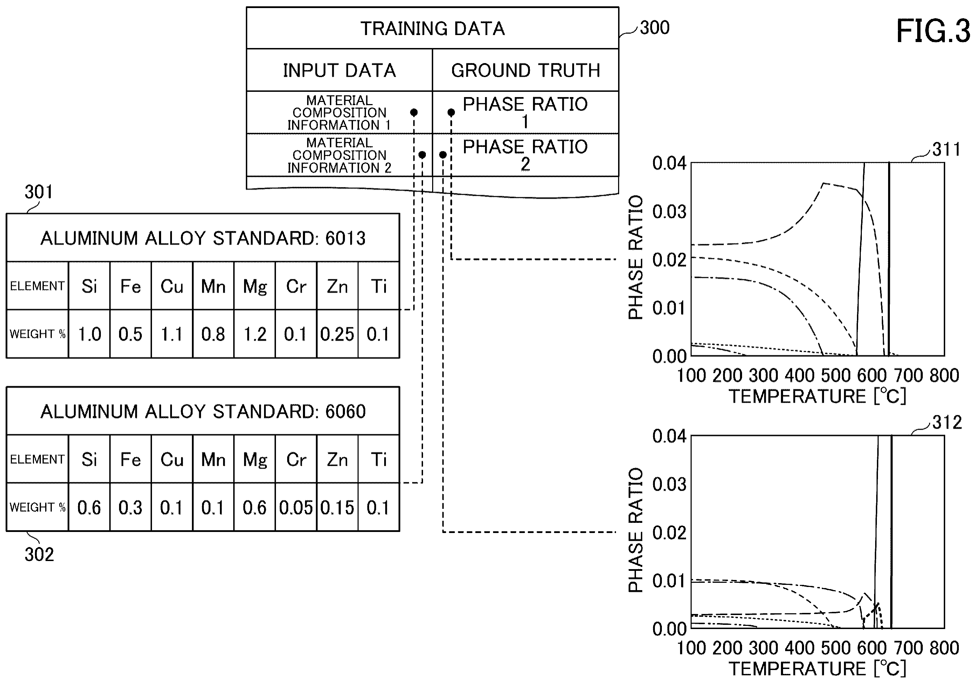

Next, a configuration of the training data, and specific examples of the input data and the ground truth included in the training data will be described. FIG. 3 is a diagram illustrating the configuration of training data and the specific examples of the input data and the ground truth.

As illustrated in FIG. 3, a training data 300 includes "input data" and "ground truth" as items of information.

The "input data" stores "material composition information 1", "material composition information 2", ... or the like, which are material composition information of different combinations of each content of each component. In FIG. 3, a reference numeral 301 indicates a specific example of the "material composition information 1", and represents an example of a content of each component, specifically, each of additive elements Si, Fe, Cu, Mn, Mg, Cr, Zn, and Ti, constituting "6013" which is a designation of the known aluminum alloy standard.

Similarly, a reference numeral 302 indicates a specific example of the "material composition information 2", and represents the content of each component, specifically, each of additive elements Si, Fe, Cu, Mn, Mg, Cr, Zn, and Ti, constituting "6060" which is a designation of a known aluminum alloy standard.

The "ground truth" stores "phase ratio 1", "phase ratio 2", ... or the like which are the phase ratios at respective temperatures within the specific temperature range associated with the "material composition information 1", "material composition information 2", ... or the like. In the example of FIG. 3, the specific temperature range is 100°C to 800°C, and 100°C and 800°C are both included in the specific temperature range.

In FIG. 3, a reference numeral 311 indicates a specific example of the "phase ratio 1", and a reference numeral 312 indicates a specific example of the "phase ratio 2", and in each specific case, the abscissa represents the temperature, and the ordinate represents the phase ratio.

The reference numeral 311 and the reference numeral 312 are results obtained by operating a simulator having a high prediction accuracy and a high cost for a long time, and in the present embodiment, these results are used as the ground truth.

The simulator described above refers to software for calculating the phase ratio at each temperature within the specific temperature range by a thermodynamic equilibrium calculation. Specifically, the relevant simulator includes software, such as CaTCalc, MatCalc, or the like. Alternatively, the relevant simulator may include software, such as Termosuite, FactStage, Pandat, or the like. Alternatively, the relevant simulator may include software, such as MALT2, Thermo-Calc, OpenCalphad, or the like.

### <Details of Each Unit of Training Device>

Next, details of each unit of the training device 110, that is, the training data generation unit 111 and the training unit 112, will be described.

### (1) Functional Configuration of Training Data Generation Unit:

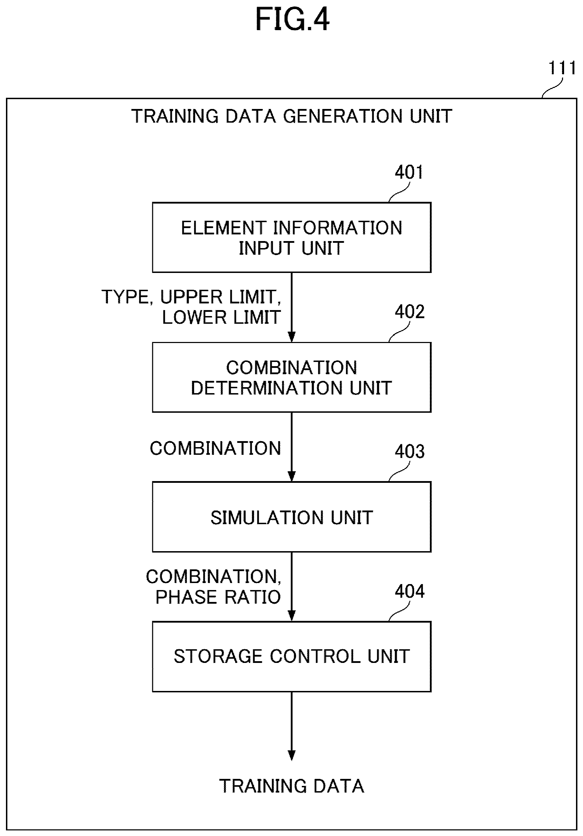

First, a functional configuration of the training data generation unit 111 will be described. FIG. 4 is a diagram illustrating an example of the functional configuration of the training data generation unit.

As illustrated in FIG. 4, the training data generation unit 111 includes an element information input unit 401, a combination determination unit 402, a simulation unit 403, and a storage control unit 404.

The element information input unit 401 receives, as each component included in the material composition information, an input of a type of an additive element to be added when generating a specific alloy, for example. The element information input unit 401 also receives an input of an upper limit value and a lower limit value of the content for each of the additive elements for which the input is received.

The combination determination unit 402 determines a plurality of combinations of the contents of the respective additive elements, by randomly selecting the contents of the respective additive elements for which the input is received, under restrictions of the upper limit value and the lower limit value. The combination of the contents of the respective additive elements may be created at predetermined increments between the upper limit value and the lower limit value. The combination determination unit 402 notifies the simulation unit 403 of the material composition information indicated by the plurality of combinations that are determined.

The simulation unit 403 calculates the phase ratio at each temperature within the specific temperature range by operating the simulator described above, for each of the plurality of material composition information notified from the combination determination unit 402. The simulation unit 403 notifies the storage control unit 404 of the plurality of material composition information, and the phase ratio at each temperature within the corresponding specific temperature range.

The storage control unit 404 generates training data in which the plurality of material composition information notified from the simulation unit 403 are set as the "input data" and the phase ratio at each temperature within the corresponding specific temperature range is set as the "ground truth". The storage control unit 404 stores the generated training data in the training data storage unit 113.

### (2) Functional Configuration of Training Unit:

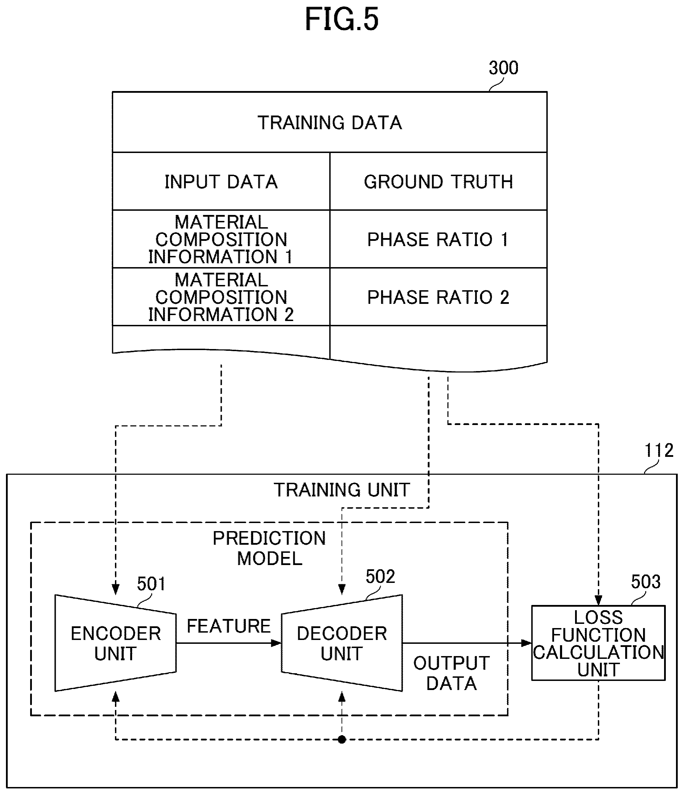

Next, a functional configuration of the training unit 112 will be described. FIG. 5 is a diagram illustrating an example of the functional configuration of the training unit.

As illustrated in FIG. 5, the training unit 112 includes a prediction model, and a loss function calculation unit 503. The prediction model is the surrogate model replacing the conventional model with machine learning. As described above, the Seq2Seq with Attention mechanism or the Transformer is implemented as the prediction model, and the prediction model includes an encoder unit 501 and a decoder unit 502.

The encoder unit 501 outputs a feature in response to receiving the "material composition information 1", the "material composition information 2", ... or the like stored in the "input data" of the training data 300.

The decoder unit 502 successively outputs output data in response to receiving the feature output from the encoder unit 501. Specifically, the decoder unit 502 reads the "phase ratio 1", the "phase ratio 2", ... or the like stored in the "ground truth" of the training data 300. Next, the decoder unit 502 outputs the output data at the (i+1)-th temperature, using the phase ratio for each of the temperatures to the i-th temperature, that is, the ground truth for each of the temperatures to the i-th temperature. Accordingly, the decoder unit 502 can successively output the output data corresponding to the phase ratio at each temperature within the specific temperature range, based for example on:
- the feature output from the encoder unit 501 in response to receiving the "material composition information 1"; and
- the "ground truth" of the "phase ratio 1". Similarly, the decoder unit 502 can successively output the output data corresponding to the phase ratio at each temperature within the specific temperature range, based for example on:
- the feature output from the encoder unit 501 in response to receiving the "material composition information 2"; and
- the "ground truth" of the "phase ratio 2".

It is assumed that a softmax function is used in an output layer of the decoder unit 502. Thus, a plurality of values, that is, values indicating the ratio of each phase, included in the output data of the decoder unit 502, are in the range of 0 to 1, and a sum of the plurality of values, that is, a sum of the values indicating the ratio of each phase, becomes "1".

Hence, by using the softmax function in the output layer of the decoder unit 502, it is possible to successively output the output data corresponding to the phase ratio at each temperature within the specific temperature range, without performing additional calculation.

The loss function calculation unit 503 reads the "phase ratio 1", the "phase ratio 2", ... or the like stored in "ground truth" of the training data 300. The loss function calculation unit 503 compares the read data with the output data at the (i+1)-th temperature output by the decoder unit 502.

The loss function calculation unit 503 calculates a plurality of types of losses when making the comparison, and calculates an integrated loss by performing a weighted addition of the plurality of calculated types of losses. In addition, the loss function calculation unit 503 updates model parameters of the encoder unit 501 and the decoder unit 502, based on the calculated integrated loss. Thus, a trained prediction model is generated. The trained prediction model in this case include a trained encoder unit and a trained decoder unit.

### (3) Operation Example 1 of Training Unit:

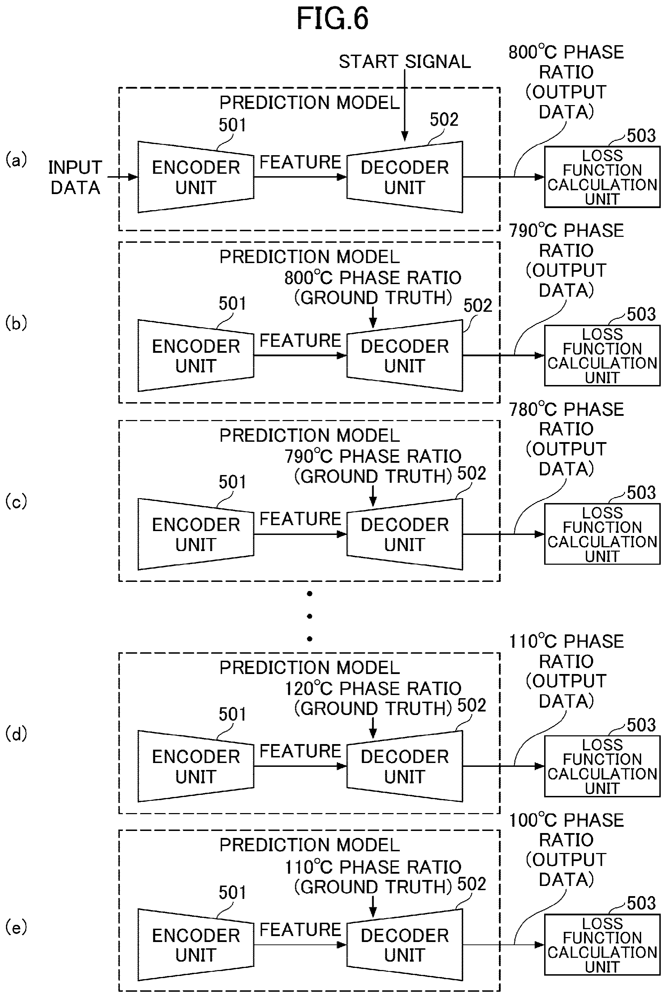

Next, an operation example of the training unit 112, mainly an operation example of the prediction model between the prediction model and the loss function calculation unit, will be described. FIG. 6 is a first diagram illustrating an operation example of the training unit.

In FIG. 6, (a) illustrates a state in which the "input data" of the training data 300 is read and input to the encoder unit 501, and the feature is output from the encoder unit 501. In FIG. 6, (a) illustrates a state in which the decoder unit 502 outputs the output data in response to the feature output from the encoder unit 501 and a start signal input to the decoder unit 502. In the case of the example in (a) of FIG. 6, the output data output by the decoder unit 502 corresponds to the phase ratio at 800°C. The output data output from the decoder unit 502, specifically, the output data corresponding to the phase ratio at 800°C, is notified to the loss function calculation unit 503. The start signal is a signal for starting the operation of the decoder unit 502, and in the present embodiment, an arbitrary value different from the output data output from the decoder unit 502, such as "000000" or the like, for example, is input to the decoder unit 502 as the start signal.

In FIG. 6, (b) illustrates a state in which the feature output from the encoder unit 501 is input to the decoder unit 502, and the ground truth at 800°C is input to the decoder unit 502, and thus, the decoder unit 502 outputs the output data. In the case of the example of (b) in FIG. 6, the output data output by the decoder unit 502 corresponds to the phase ratio at 790°C. The output data output from the decoder unit 502, specifically, the output data corresponding to the phase ratio at 790°C, is notified to the loss function calculation unit 503.

In FIG. 6, (c) illustrates a state in which the feature output from the encoder unit 501 is input to the decoder unit 502, and the ground truth of the phase ratio for the temperatures to 790°C is input to the decoder unit 502, and thus, the decoder unit 502 outputs the output data. However, in (c) of FIG. 6, only the ground truth at 790°C is illustrated for the sake of convenience due to limited space. Actually, the ground truth at 800°C and the ground truth at 790°C are input to the decoder unit 502 with weighting.

In the example illustrated in (c) of FIG. 6, the output data output from the decoder unit 502 corresponds to the phase ratio at 780°C. The output data output from the decoder unit 502, specifically, the output data corresponding to the phase ratio at 780°C, is notified to the loss function calculation unit 503.

In FIG. 6, (d) illustrates a state in which the feature output from the encoder unit 501 is input to the decoder unit 502, and the ground truth of the phase ratio for the temperatures to 120°C is input to the decoder unit 502, and thus, the decoder unit 502 outputs the output data. However, in (d) of FIG. 6, only the ground truth at 120°C is illustrated for the sake of convenience due to limited space. Actually, each ground truth from 800°C to 120°C is input to the decoder unit 502 with weighting.

In the example illustrated in (d) of FIG. 6, the output data output from the decoder unit 502 corresponds to the phase ratio at 110°C. The output data output from the decoder unit 502, specifically, the output data corresponding to the phase ratio at 110°C, is notified to the loss function calculation unit 503.

In FIG. 6, (e) illustrates a state in which the feature output from the encoder unit 501 is input to the decoder unit 502, and ground truth of the phase ratio for the temperatures to 110°C is input to the decoder unit 502, and thus, the decoder unit 502 outputs the output data. However, in (e) of FIG. 6, only the ground truth at 110°C is illustrated for the sake of convenience due to limited space. Actually, each ground truth from 800°C to 110°C are input to the decoder unit 502 with weighting.

In the example illustrated in (e) of FIG. 6, the output data output from the decoder unit 502 corresponds to the phase ratio at 100°C. The output data output from the decoder unit 502, specifically, the output data corresponding to the phase ratio at 100°C, is notified to the loss function calculation unit 503.

### (4) Operation Example 2 of Training Unit:

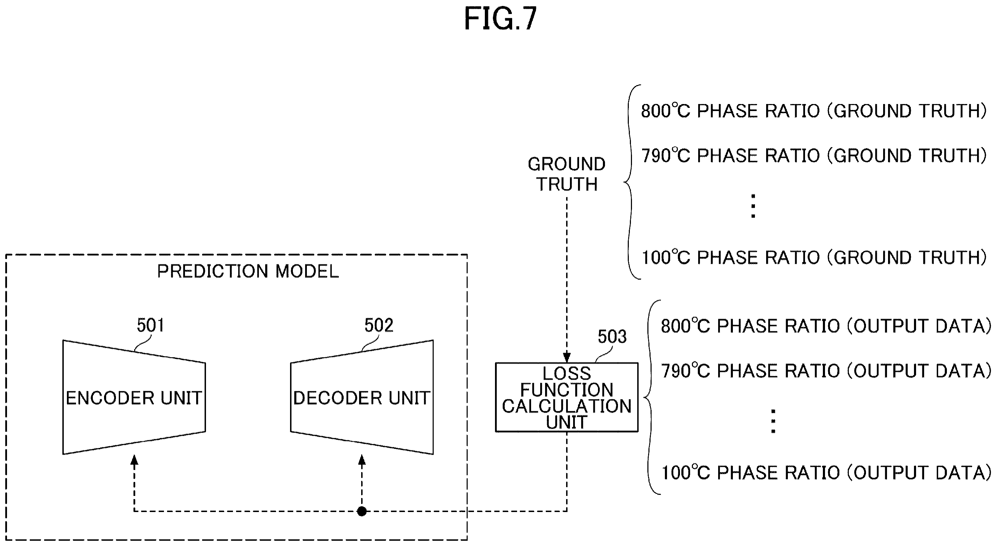

Next, an operation example of the training unit 112, mainly an operation example of the loss function calculation unit 503 between the prediction model and the loss function calculation unit, will be described. FIG. 7 is a second diagram illustrating the operation example of the training unit. As illustrated in FIG. 7, the loss function calculation unit 503 reads the "ground truth" of the training data 300. The example illustrated in FIG. 7 illustrates a state in which:
- the ground truth of the phase ratio at 800°C;
- the ground truth of the phase ratio at 790°C; ...
- the ground truth of the phase ratio at 100°C, which are the phase ratio at each temperature within the specific temperature range included in the "phase ratio 1".

In the example of FIG. 7 illustrates a state in which, as a result of successively outputting the output data from the decoder unit 502, based on the feature output from the encoder unit 501 in response to the input of the "material composition information 1":
- the output data of the phase ratio at 800°C;
- the output data of the phase ratio at 790°C;
   ...
- the output data of the phase ratio at 100°C,
are held in the loss function calculation unit 503.

The example of FIG. 7 illustrates a state in which the loss function calculation unit 503 compares the ground truth of the phase ratio at each of the temperatures of 100°C to 800°C with the output data of the phase ratio at each of the temperatures of 100°C to 800°C, and calculates the integrated loss. The example of FIG. 7 illustrates a state in which the loss function calculation unit 503 updates the model parameters of the encoder unit 501 and the decoder unit 502, based on the calculated integrated loss. The functional configuration of the loss function calculation unit 503 for calculating the integrated loss will be described below in more detail.

### (5) Details of Functional Configuration of Loss Function Calculation Unit:

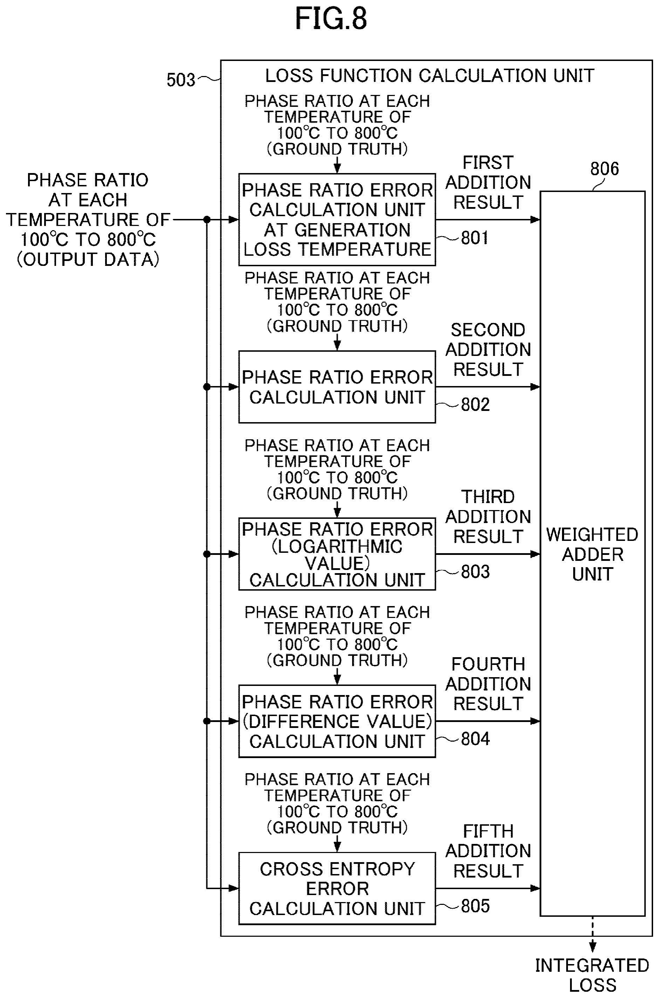

FIG. 8 is a diagram illustrating an example of a method of calculating the integration loss by the loss function calculation unit. As illustrated in FIG. 8, the loss function calculation unit 503 includes, as functions for calculating a plurality of types of losses:
- a phase ratio error calculation unit 801 at a generation/disappearance temperature;
- a phase ratio error calculation unit 802;
- a phase ratio error (logarithmic value) calculation unit 803;
- a phase ratio error (difference value) calculation unit 804;
- a cross entropy error calculation unit 805; and
- a weighted adder unit 806.

The phase ratio error calculation unit 801 at the generation/loss temperature specifies the temperature at which the curve of each phase ratio becomes 0 in the ground truth of the training data. The phase ratio error calculation unit 801 at the generation/loss temperature compares the ground truth of the phase ratio at each of the temperatures of 100°C to 800°C and the output data of the phase ratio at each of the temperatures of 100°C to 800°C, with respect to the specified temperature. Thus, the phase ratio error calculation unit 801 at the generation/loss temperature adds the error between the phase ratio at the temperature at which each phase specified based on the training data is generated or lost, and the phase ratio specified based on the training data, for all of the phases. The phase ratio error calculation unit 801 at the generation/loss temperature outputs a first addition result of the addition for all of the phases.

The phase ratio error calculation unit 802 compares the ground truth of the phase ratio at each of the temperatures of 100°C to 800°C with the output data of the phase ratio at each of the temperatures of 100°C to 800°C. Accordingly, the phase ratio error calculation unit 802 adds the error between the phase ratios at each temperature within the specific temperature range, and outputs a second addition result.

The phase ratio error (logarithmic value) calculation unit 803 compares the ground truth of the phase ratio at each of the temperatures of 100°C to 800°C with the output data of the phase ratio at each of the temperatures of 100°C to 800°C. Accordingly, the phase ratio error (logarithmic value) calculation unit 803 adds the error between logarithmic values of the phase ratios at each temperature within the specific temperature range, and outputs a third addition result.

The phase ratio error (difference value) calculation unit 804 compares the ground truth of the phase ratio at each of the temperatures of 100°C to 800°C with the output data of the phase ratio at each of the temperatures of 100°C to 800°C. Accordingly, the phase ratio error (difference value) calculation unit 804 adds the error between difference values of the phase ratios at adjacent temperatures within the specific temperature range, and outputs a fourth addition result.

The cross entropy error calculation unit 805 compares the ground truth of the phase ratio at each of the temperatures of 100°C to 800°C with the output data of the phase ratio at each of the temperatures of 100°C to 800°C. Accordingly, the cross entropy error calculation unit 805 adds the error between ratios of the phase ratios at each temperature within the specific temperature range, and outputs a fifth addition result.

The weighted adder unit 806 calculates the integrated loss by weighting and adding the first addition result through the fifth addition result output from the phase ratio error calculation unit 801 at the generation/loss temperature through the cross entropy error calculation unit 805.

Accordingly, by calculating the plurality of types of losses and performing the weighted addition, the loss function calculation unit 503 can process the loss between the output data and the ground truth from various perspectives. As a result, the training unit 112 can appropriately update the model parameters when training the prediction model.

### <Flow of Training Process>

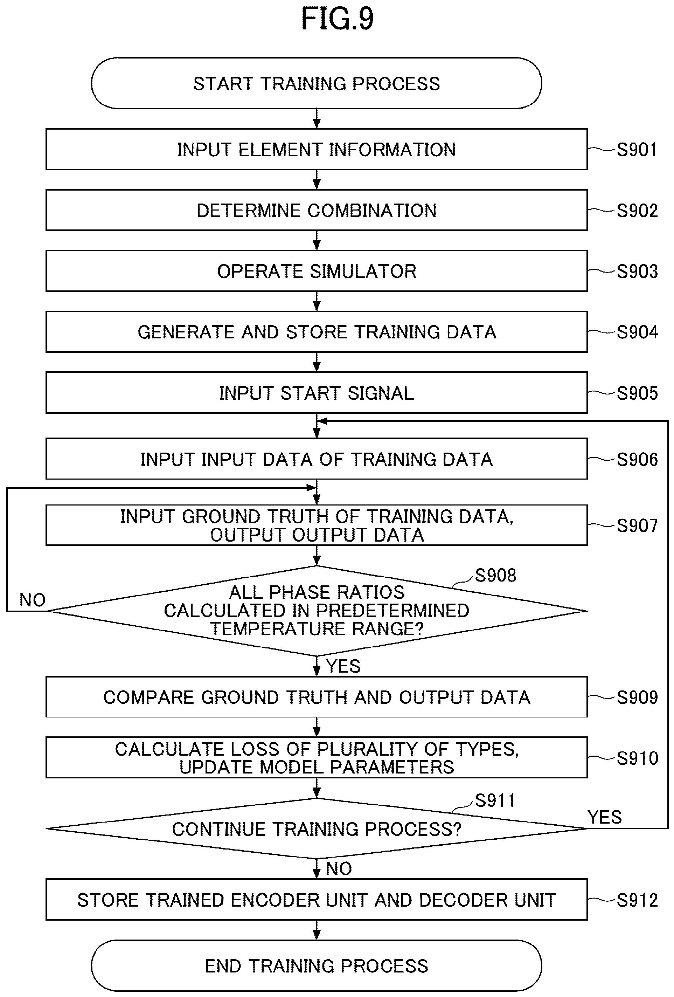

Next, a flow of a training process performed by the training device 110 will be described. FIG. 9 is a flow chart illustrating the flow of the training process.

In step S901, the training data generation unit 111 receives, as the element information, input of the types of additive elements to be added when generating a specific alloy, and the upper limit value and the lower limit value of the content of each of the additive elements.

In step S902, the training data generation unit 111 determines a plurality of combinations of the contents of the respective additive elements, by randomly selecting the contents of the respective additive elements under the restrictions of the upper limit value and the lower limit value.

In step S903, the training data generation unit 111 operates the simulator for each of the material composition information indicated by the plurality of combinations that are determined, and calculates the phase ratio at each temperature within the specific temperature range.

In step S904, the training data generation unit 111 generates the training data, and thereafter stores the generated training data in the training data storage unit 113.

In step S905, the training unit 112 inputs the start signal to the decoder unit 502.

In step S906, the training unit 112 reads the "input data" of the training data, and thereafter inputs the input data to the prediction model.

In step S907, the training unit 112 successively inputs the phase ratio for each of the temperatures to the i-th temperature of the "ground truth" of the training data, that is, the ground truth for each of the temperatures to the i-th temperature, and successively outputs the (i+1)-th output data.

In step S908, the training unit 112 determines whether or not all of the output data corresponding to the phase ratio at each temperature within the specific temperature range are output. If it is determined in step S908 that there is a temperature for which the output data corresponding to the phase ratio is not output (when NO in step S908), the process returns to step S907.

On the other hand, if it is determined in step S908 that all of the output data corresponding to the phase ratio at each temperature within the specific temperature range are output (when YES in step S908), the process proceeds to step S909.

In step S909, the training unit 112 reads the "ground truth" of the training data, and thereafter compares the read ground truth with the output data.

In step S910, the training unit 112 calculates the plurality of types of losses, and thereafter performs the weighted addition of the plurality of types of losses that are calculated, in order to calculate the integrated loss. The training unit 112 updates the model parameters of the encoder unit 501 and the decoder unit 502, based on the calculated integrated loss.

In step S911, the training unit 112 determines whether or not to continue the training process. If it is determined in step S911 that the training process is to be continued (when YES in step S911), the process returns to step S906, and the same process is performed by reading the next "input data" of the training data.

On the other hand, if it is determined in step S911 that the training process is to be ended (when NO in step S911), the process proceeds to step S912.

In step S912, the training unit 112 stores the trained encoder unit and the trained decoder unit in the storage unit of the prediction device 120 and the storage unit of the prediction result analysis device 130, as the trained prediction model.

### <Details of Each Unit of Prediction Device>

Next, the prediction unit 122 of the prediction device 120 will be described in more detail.

### (1) Functional Configuration of Prediction Unit:

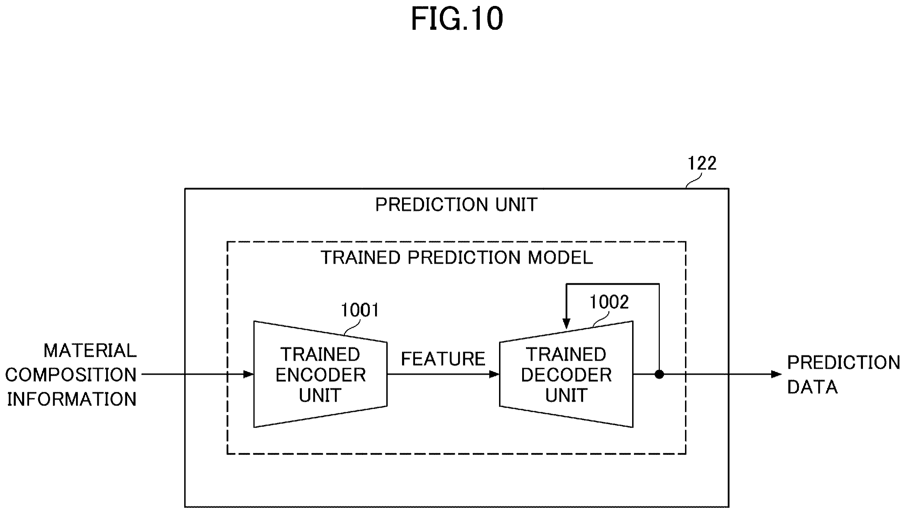

First, a functional configuration of the prediction unit 122 will be described. FIG. 10 is a diagram illustrating an example of the functional configuration of the prediction unit.

As illustrated in FIG. 10, the prediction unit 122 includes a trained encoder unit 1001 and a trained decoder unit 1002, which are already trained by the training unit 112. The trained encoder unit 1001 and the trained decoder unit 1002 form a trained prediction model.

The trained encoder unit 1001 calculates a feature in response to receiving the material composition information of the material to be predicted, notified from the material composition input unit 121, and thereafter outputs the calculated feature to the trained decoder unit 1002.

The trained decoder unit 1002 successively outputs the prediction data in response to receiving the feature output from the trained encoder unit 1001. Specifically, the trained decoder unit 1002 outputs the prediction data at the (i+1)-th temperature, using prediction data at each of the temperatures to the i-th temperature. Thus, the trained decoder unit 1002 can predict the phase ratio at each temperature within the specific temperature range, based on the feature output from the trained encoder unit 1001 in response to the input of the material composition information of the material to be predicted.

### (2) Operation Example of Prediction Unit:

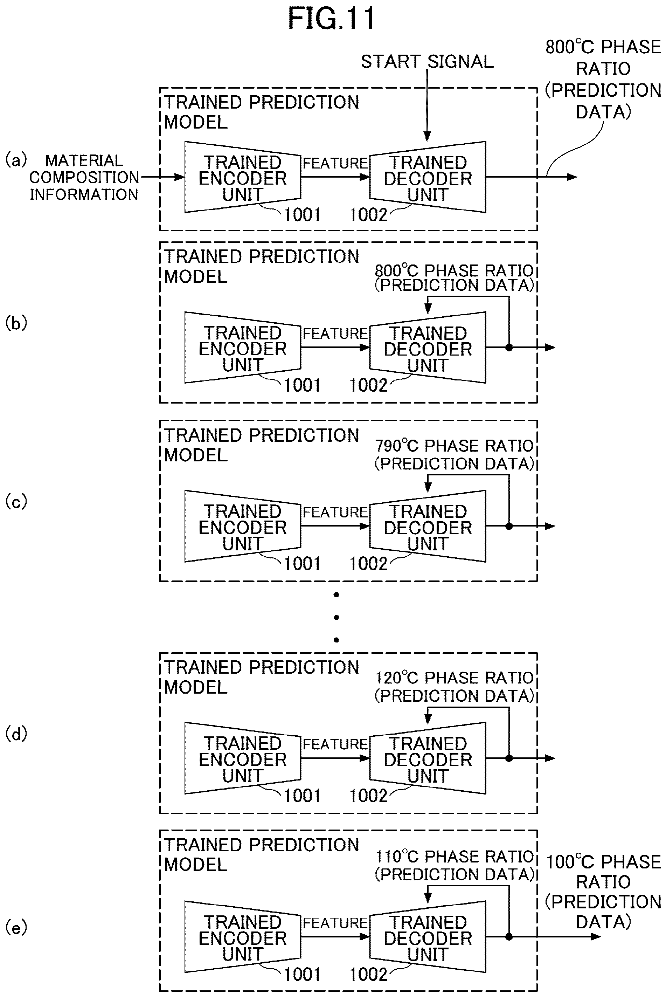

Next, an operation example of the prediction unit 122 will be described. FIG. 11 is a first diagram illustrating the operation example of the prediction unit. In FIG. 11, (a) illustrates a state in which the material composition information of the material to be predicted is input to the trained encoder unit 1001, and thus, the feature is output from the trained encoder unit 1001. In addition, (a) of FIG. 11 illustrates a state in which the feature output from the trained encoder unit 1001 is input to the trained decoder unit 1002, and the start signal is input to the trained decoder unit 1002, and thus, the phase ratio at 800°C is output.

(b) of FIG. 11 illustrates a state in which the feature output from the trained encoder unit 1001 is input to the trained decoder unit 1002, and the phase ratio at 800°C, that is, the prediction data, is input to the trained decoder unit 1002. Thus, the trained decoder unit 1002 outputs the phase ratio at 790°C as the prediction data.

(c) of FIG. 11 illustrates a state in which the feature output from the trained encoder unit 1001 is input to the trained decoder unit 1002, and the phase ratio at temperatures to 790°C, that is, the prediction data, is input to the trained decoder unit 1002. In (c) of FIG. 11, only the phase ratio at 790°C is illustrated for the sake of convenience due to limited space. Actually, the phase ratio at 800°C and the phase ratio at 790°C are input to the trained decoder unit 1002 with weighting. Thus, the trained decoder unit 1002 outputs the phase ratio at 780°C, as the prediction data.

(d) of FIG. 11 illustrates a state in which the feature output from the trained encoder unit 1001 is input to the trained decoder unit 1002, and the phase ratio at temperatures to 120°C, that is, the prediction data, is input to the trained decoder unit 1002. In (d) of FIG. 11, only the phase ratio at 120°C is illustrated for the sake of convenience due to limited space. Actually, the phase ratio at each of the temperatures of 800°C to 120°C is input to the trained decoder unit 1002 with weighting. Thus, the trained decoder unit 1002 outputs the phase ratio at 110°C, as the prediction data.

(e) of FIG. 11 illustrates a state in which the feature output from the trained encoder unit 1001 is input to the trained decoder unit 1002, and the phase ratio to the temperature of 110°C, that is, the prediction data, is input to the trained decoder unit 1002. In (e) of FIG. 11, only the phase ratio at 110°C is illustrated for the sake of convenience due to limited space. Actually, the phase ratio at each of the temperatures of 800°C to 110°C is input to the trained decoder unit 1002 with weighting. Thus, the example of (e) of FIG. 11 also illustrates the phase ratio at 100°C, that is, the prediction data, output from the trained decoder unit 1002 in response to the input of the feature value and the phase ratio to the temperature of 110°C, that is, the prediction data.

### <Flow of Prediction Process>

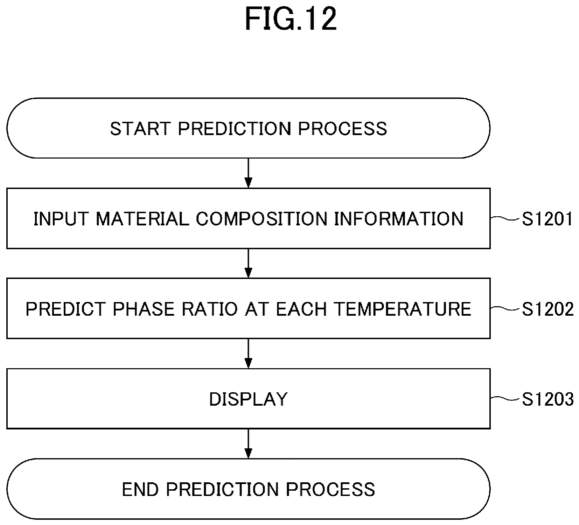

Next, a flow of a prediction process performed by the prediction device 120 will be described. FIG. 12 is a flow chart illustrating the flow of the prediction process.

In step S1201, the material composition input unit 121 receives an input of the material composition information of the material to be predicted.

In step S1202, the prediction unit 122 receives the start signal, and inputs the input material composition information to the trained prediction model, thereby predicting the phase ratio at each temperature within the specific temperature range.

In step S1203, the display unit 123 displays the predicted phase ratio at each temperature.

### <Prediction Accuracy of Phase Fraction>

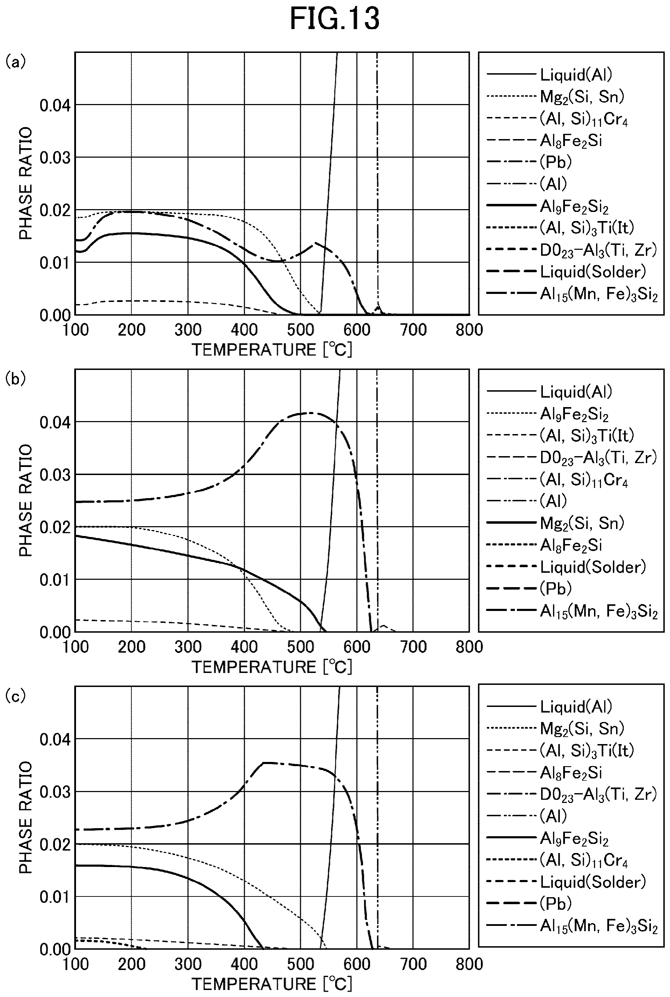

Next, a prediction accuracy of the phase ratio at each temperature within the specific temperature range predicted by the prediction unit 122 will be described. FIG. 13 is a diagram for explaining the prediction accuracy of the phase ratio. In FIG. 13, each of (a) through (c) illustrates the prediction data for a case where the content of each additive element constituting "6013", which is the designation of the known aluminum alloy standard, is input as the material composition information of the material to be predicted. In the present embodiment, the ground truth indicated by reference numeral 311 in FIG. 3 is used to evaluate the prediction accuracy. The loss from the ground truth is calculated using a mean squared logarithmic error (MSLE) loss.

In FIG. 13, (a) illustrates the prediction data for a case where a fully connected multilayer neural network is applied to the prediction model, as a comparative example. In (a) of FIG. 13, the MSLE loss from the ground truth is 3.79 × 10⁻⁴.

In FIG. 13, (b) illustrates the prediction data for a case where the Seq2Seq with the Attention mechanism is applied to the prediction model. In (b) of FIG. 13, the MSLE loss from the ground truth is 5.10 × 10⁻⁵.

In FIG. 13, (c) illustrates the prediction data for a case where the Transformer is applied to the prediction model. In (c) of FIG. 13, the MSLE loss from the ground truth is 2.45 × 10⁻⁵.

As described above, when the fully connected multilayer neural network is applied to the prediction model in order to calculate the phase ratio at a low cost, the prediction accuracy was low in some temperature regions, such as regions from 400°C to 600°C, for example.

In contrast, when the Seq2Seq with Attention mechanism is applied to the prediction model in order to calculate the phase ratio at a low cost, it was possible to significantly improve the loss. Further, when Transfomer is applied to the prediction model, it was possible to reproduce the phase ratio of substantially the same level as the ground truth.

That is, when predicting the phase ratio over the specific temperature range based on the material composition information, the prediction accuracy can be improved by:
- processing the phase ratio at each temperature as time series data, using a recurrent neural network; and
- processing the loss from various perspectives using a plurality of types of loss functions.

### <Details of Prediction Result Analysis Device>

Next, as details of the prediction result analysis device 130, specific examples of processes of the material composition input unit 131, the baseline composition input unit 132, the intermediate composition generation unit 133, and the integral gradient calculation unit 135, and a functional configuration and an operation example of the prediction unit 134 will be described.

### (1) Specific Examples of Processes of Material Composition Input Unit, Baseline Composition Input Unit, and Intermediate Composition Generation Unit:

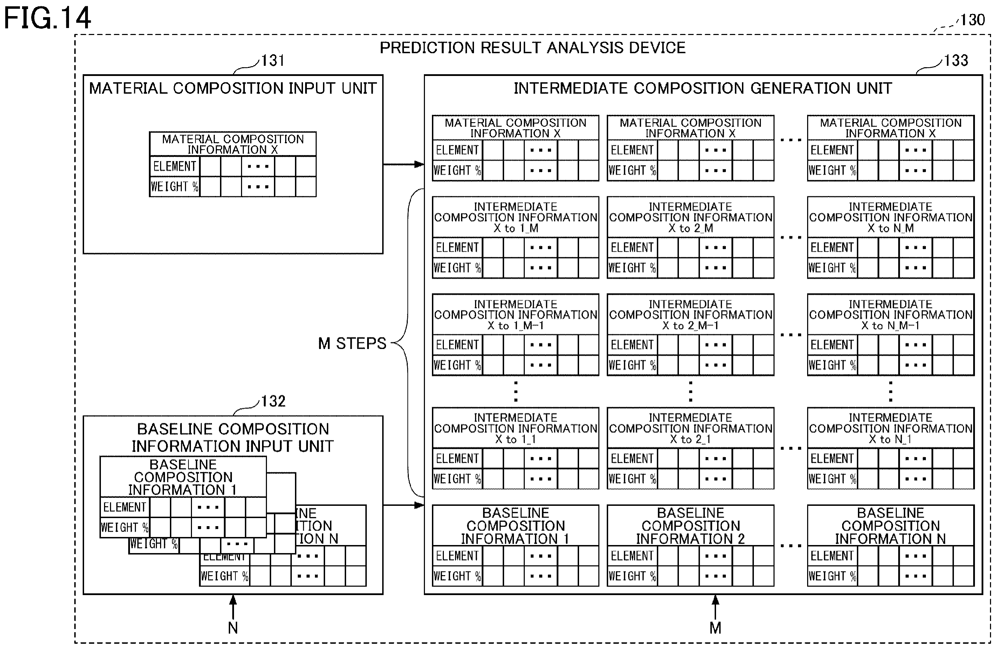

First, the specific examples of the processes of the material composition input unit 131, the baseline composition input unit 132, and the intermediate composition generation unit 133, among the units included in the prediction result analysis device 130, will be described. FIG. 14 is a first diagram illustrating the specific example of the process of the prediction result analysis device.

As illustrated in FIG. 14, the material composition input unit 131 receives an input of "material composition information X", as the material composition information of the material to be predicted. The baseline composition input unit 132 receives an input of a number "N" of baseline composition information, and receives an input of a plurality of baseline composition information, specifically, "baseline composition information 1" through "baseline composition information N" which are N baseline composition information.

The intermediate composition generation unit 133 generates M intermediate composition information by dividing between the material composition information of the material to be predicted and each of the N baseline composition information by a predetermined number of steps = M. The example of FIG. 14 illustrates:
- a state where "intermediate composition information X to 1_1" through "intermediate composition information X to 1_M" are generated by dividing between the "material composition information X" and "baseline composition information 1" by the number of steps = M;
- a state where "intermediate composition information X to 2_1" through "intermediate composition information X to 2_M" are generated by dividing between the "material composition information X" and the "baseline composition information 2" by the number of steps = M; ...
- a state where "intermediate composition information X to N_1" through "intermediate composition information X to N_M" are generated by dividing between the "material composition information X" and the "baseline composition information N" by the number of steps = M.

### (2) Functional Configuration of Prediction Unit:

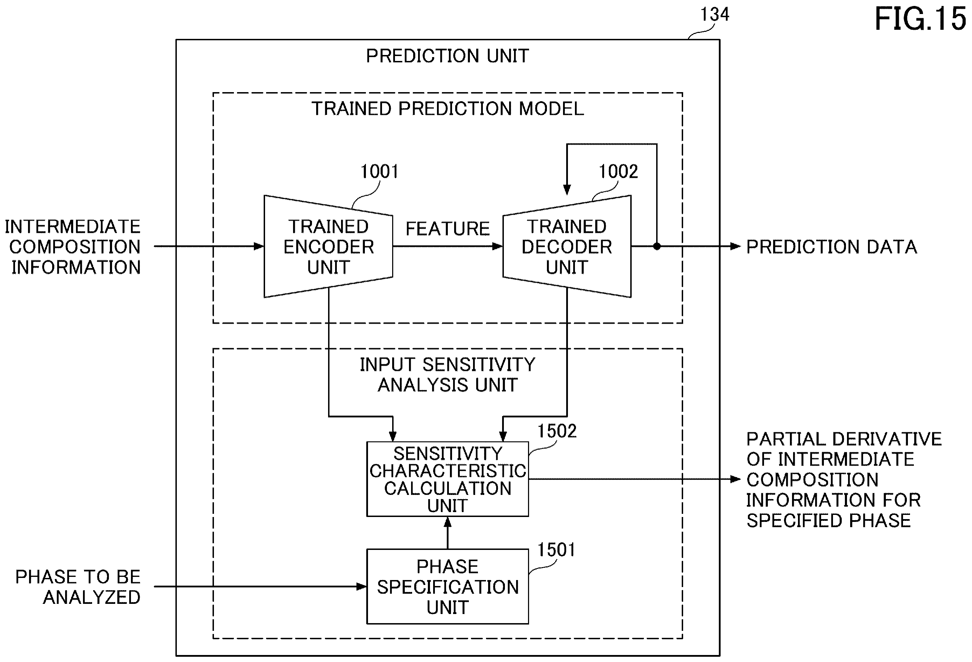

Next, the functional configuration of the prediction unit 134 will be described in detail. FIG. 15 is a second diagram illustrating an example of the functional configuration of the prediction unit.

As illustrated in FIG. 15, the prediction unit 134 includes a trained encoder unit 1001 and a trained decoder unit 1002, which are already trained by the training unit 112. The trained encoder unit 1001 and the trained decoder unit 1002 form a trained prediction model.

The functions of the trained encoder unit 1001 and the trained decoder unit 1002 are the same as the functions of the trained encoder unit 1001 and the trained decoder unit 1002 of FIG. 10, except that the intermediate composition information is input in place of the material composition information of the material to be predicted. Accordingly, a description thereof will be omitted.

As illustrated in FIG. 15, the prediction unit 134 further includes a phase specification unit 1501 and a sensitivity characteristic calculation unit 1502. The phase specification unit 1501 and the sensitivity characteristic calculation unit 1502 form an input sensitivity analysis unit.

The phase specification unit 1501 receives an input of the phase to be analyzed, specified by the designer of the material design. The phase to be analyzed, specified by the designer of the material design, refers to a phase for which the designer of the material design desires to understand in advance a varying extent at each temperature when each component included in the material composition is modified. The phase specification unit 1501 notifies the sensitivity characteristic calculation unit 1502 of the input phase to be analyzed, received by the phase specification unit 1501.

When the prediction data at the (i+1)-th temperature is output, the sensitivity characteristic calculation unit 1502 derives a partial derivative by partially differentiating the value predicted for the phase notified from the phase specification unit 1501, using the intermediate composition information notified from the intermediate composition generation unit 133.

Specifically, the sensitivity characteristic calculation unit 1502 uses:
- the partial derivative in each layer, calculated by performing a process in each layer within the trained encoder unit 1001, during a period from a time when the intermediate composition information is input until a time when the feature is output; and
- the partial derivative in each layer, calculated by performing a process in each layer within the trained decoder unit 1002, during a period from a time when the feature is input until a time when a value predicted for a specified phase is output at the (i+1)-th temperature, to derive a "partial derivative obtained by partially differentiating a value at the (i+1)-th temperature, predicted for the specified phase to be analyzed, using the intermediate composition information".

In addition, the sensitivity characteristic calculation unit 1502 notifies the integral gradient calculation unit 135 of the "partial derivative obtained by partially differentiating a value at the (i+1)-th temperature, predicted for the specified phase to be analyzed, using the intermediate composition information".

### (3) Operation Example of Prediction Unit:

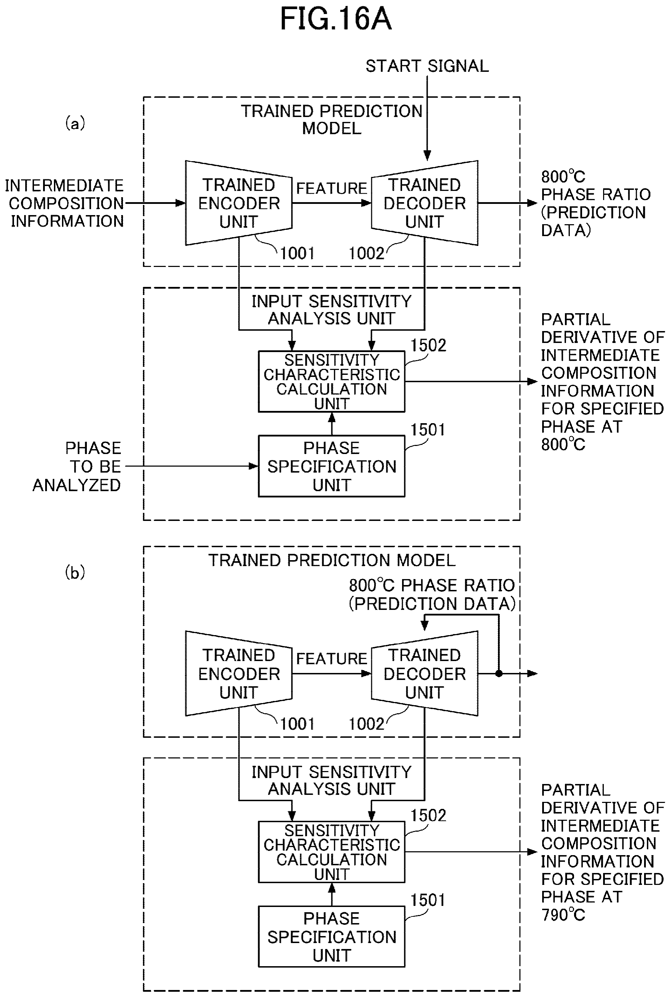

Next, a specific example of the process of the prediction unit 134 will be described. FIG. 16A through FIG. 16C are second through fourth diagrams illustrating operation examples of the prediction unit. In FIG. 16A, (a) illustrates a state in which the feature is output from the trained encoder unit 1001, in response to the input of the intermediate composition information, generated based on the material composition information of the material to be predicted and the baseline composition information, to the trained encoder unit 1001. In addition, (a) of FIG. 16A illustrates a state in which the phase ratio at 800°C is output, in response to the input of the feature output from the trained encoder unit 1001 to the trained decoder unit 1002, and the input of the start signal to the trained decoder unit 1002.

Further, in FIG. 16A, (a) illustrates a state in which, when the phase ratio at 800°C is output as prediction value, a "partial derivative obtained by partially differentiating a value predicted for the specified phase, using the intermediate composition information" is derived for each component at 800°C.

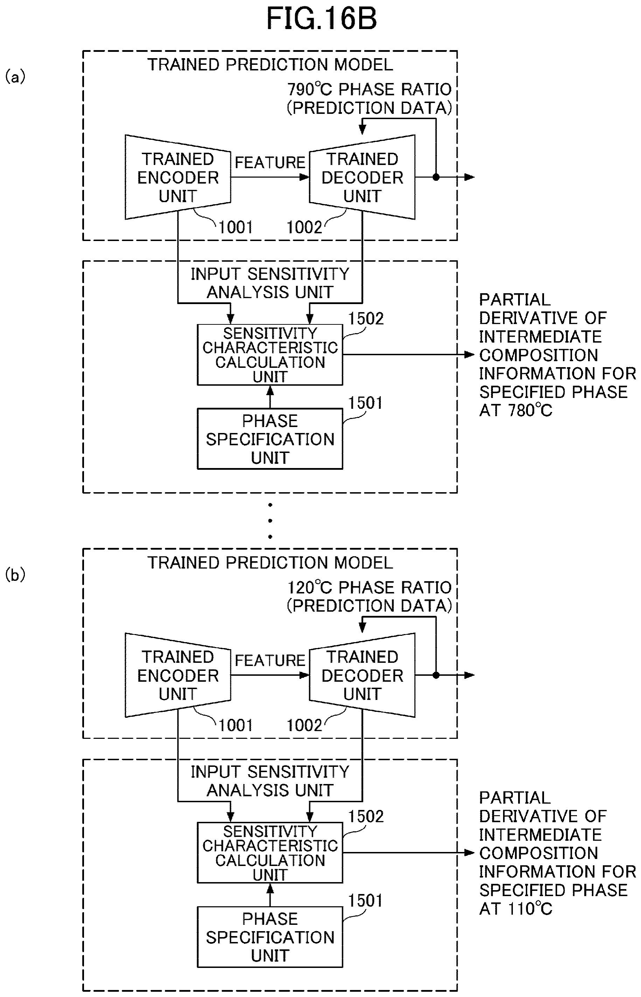

In FIG. 16A, (b) illustrates a state in which the feature output from the trained encoder unit 1001 is input to the trained decoder unit 1002, and the phase ratio at 800°C, that is the prediction data, is input to the trained decoder unit 1002. Thus, the trained decoder unit 1002 outputs the phase ratio at 790°C, as the prediction data.

Further, (b) of FIG. 16A illustrates a state in which the "partial derivative obtained by partially differentiating a value predicted for the specified phase, using the intermediate composition information" is derived for each component at 790°C, when the phase ratio at 790°C is output as the prediction value.

In FIG. 16B, (a) illustrates a state in which the feature output from the trained encoder unit 1001 is input to the trained decoder unit 1002, and the phase ratio to 790°C is input to the trained decoder unit 1002. In (a) of FIG. 16B, only the phase ratio at 790°C is illustrated for the sake of convenience due to limited space. Actually, the phase ratio at 800°C and the phase ratio at 790°C are input to the trained decoder unit 1002 with weighting. Thus, the trained decoder unit 1002 outputs the phase ratio at 780°, as the prediction data.

Further, (a) of FIG. 16B illustrates a state in which the "partial derivative obtained by partially differentiating a value predicted for the specified phase, using the intermediate composition information" is derived for each component at 780°C, when the phase ratio at 780°C is output as the prediction value.

In FIG. 16B, (b) illustrates a state in which the feature output from the trained encoder unit 1001 is input to the trained decoder unit 1002, and the phase ratio to 120°C is input to the trained decoder unit 1002. In (b) of FIG. 16B, only the phase ratio at 120°C is illustrated for the sake of convenience due to limited space. Actually, the phase ratio at each of the temperatures of 800°C to 120°C is input to the trained decoder unit 1002 with weighting. Thus, the trained decoder unit 1002 outputs the phase ratio at 110°, as the prediction data.

Moreover, in FIG. 16B, (b) illustrates a state in which the "partial derivative obtained by partially differentiating a value predicted for the specified phase, using the intermediate composition information" is derived for each component at 110°C, when the phase ratio at 110°C is output as the prediction value.

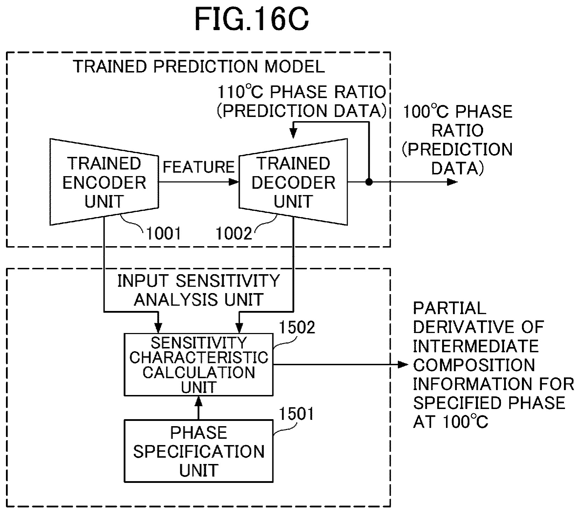

FIG. 16C illustrates a state in which the feature output from the trained encoder unit 1001 is input to the trained decoder unit 1002, and the phase ratio to 110°C, which is the prediction data, is input to the trained decoder unit 1002. In FIG. 16C, only the phase ratio at 110°C is illustrated for the sake of convenience due to limited space. Actually, the phase ratios at each of the temperatures of 800°C to 110°C is input to the trained decoder unit 1002 with weighting. Thus, the trained decoder unit 1002 outputs the phase ratio at 100°, as the prediction data. The example of FIG. 16C also illustrates the phase ratio at 100°C, which is the prediction data output from the trained decoder unit 1002 in response to the input of the feature value and the phase ratio to 110°C.

Further, FIG. 16C illustrates a state in which the "partial derivative obtained by partially differentiating a value predicted for the specified phase, using the intermediate composition information" is derived for each component at 100°C, when the phase ratio at 100°C is output as the prediction value.

### (4) Specific Examples of Processes of Prediction Unit and Integral Gradient Calculation Unit:

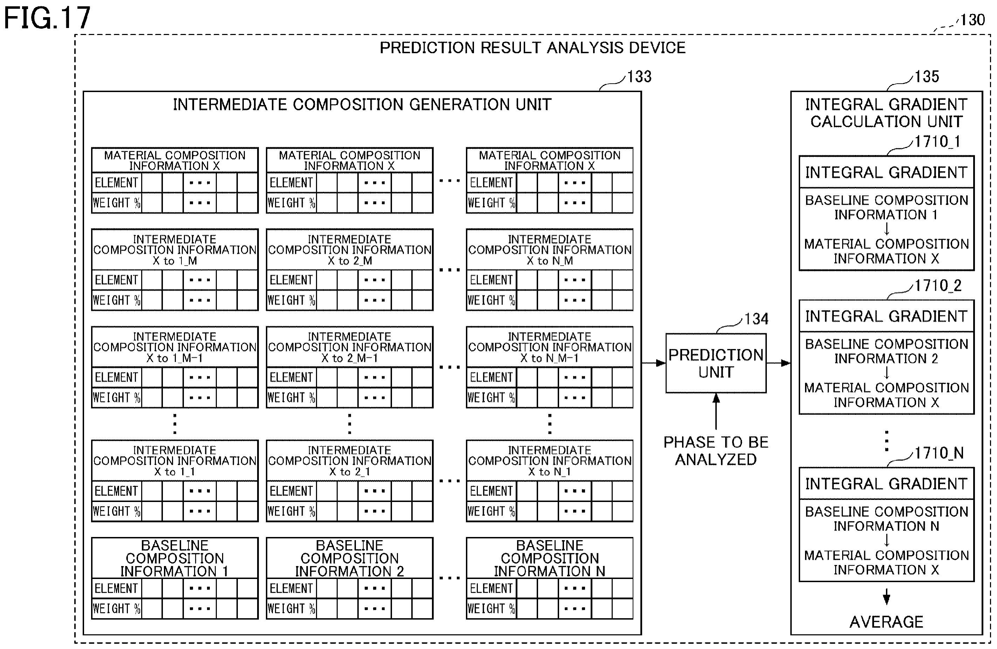

Next, specific examples of processes of the prediction unit 134 and the integral gradient calculation unit 135, among the units included in the prediction result analysis device 130, will be described. FIG. 17 is a second diagram illustrating a specific example of the process of the prediction result analysis device.

As illustrated in FIG. 17, the prediction unit 134 successively predicts M phase ratios at each temperature within the specific temperature range, by inputting the M intermediate composition information notified from the intermediate composition generation unit 133 to the trained prediction model. In addition, the prediction unit 134 derives M partial derivatives for each temperature and each component with respect to the phase to be analyzed.

According to the example of FIG. 17, the prediction unit 134 performs a process including:
- predicting M phase ratios for the "baseline composition information 1", in response to the input of M intermediate composition information from "intermediate composition information X to 1_1" through "intermediate composition information X to 1_M", and further, deriving M partial derivatives for each temperature and each component with respect to the phase to be analyzed;
- predicting M phase ratios for the "baseline composition information 2", in response to the input of M intermediate composition information from "intermediate composition information X to 2_1" through "intermediate composition information X to 2_M", and further, deriving M partial derivatives for each temperature and each component with respect to the phase to be analyzed; ...
- predicting M phase ratios for the "baseline composition information N", in response to the input of M intermediate composition information from "intermediate composition information X to N_1" through "intermediate composition information X to N_M", and further, deriving M partial derivatives for each temperature and each component with respect to the phase to be analyzed.

As illustrated in FIG. 17, the integral gradient calculation unit 135 calculates the integral gradient of each temperature and each component, using the M partial derivatives derived for each temperature and each component by the prediction unit 134.

The example of FIG. 17 illustrates the integral gradient calculation unit 135 in a state where:
- the partial derivative of each temperature and each component, derived for the "intermediate information X to 1_M", is acquired;
- the partial derivative of each temperature and each component, derived for the "intermediate information X to 1_M-1" , is acquired; ...
- the partial derivative of each temperature and each component, derived for the "intermediate information X to 1_1" , is acquired; and
- the M partial derivatives are integrated using a composition weight calculated in advance for each temperature and each component, and an integral gradient 1710_1 is thereafter calculated by averaging the integrated partial derivatives.

Similarly, the example of FIG. 17 illustrates the integral gradient calculation unit 135 in a state where:
- the partial derivative of each temperature and each component, derived for the "intermediate composition information X to 2_M", is acquired;
- the partial derivative of each temperature and each component, derived for the "intermediate composition information X to 2_M-1", is acquired; ...
- the partial derivative of each temperature and each component, derived for the "intermediate composition information X to 2_1", is acquired; and
- the M partial derivatives are integrated using a composition weight calculated in advance for each temperature and each component, and an integral gradient 1710_2 is thereafter calculated by averaging the integrated partial derivatives.

Similarly, the example of FIG. 17 illustrates the integral gradient calculation unit 135 in a state where:
- the partial derivative of each temperature and each component, derived for the "intermediate composition information X to N_M", is acquired;
- the partial derivative of each temperature and each component, derived for the "intermediate composition information X to N_M-1", is acquired; ...
- the partial derivative of each temperature and each component, derived for the "intermediate composition information X to N_1", is acquired; and
- the M partial derivatives are integrated using a composition weight calculated in advance for each temperature and each component, and an integral gradient 1710_N is thereafter calculated by averaging the integrated partial derivatives.

Further, the example of FIG. 17 illustrates a state where the integral gradient calculation unit 135 calculates a final integral gradient with respect to the phase to be analyzed, that is, the integral gradient after the averaging, by calculating the average value of the plurality of integral gradients 1710_1 through 1710_N for each temperature and each component.

### <Flow of Prediction Result Analysis Process>

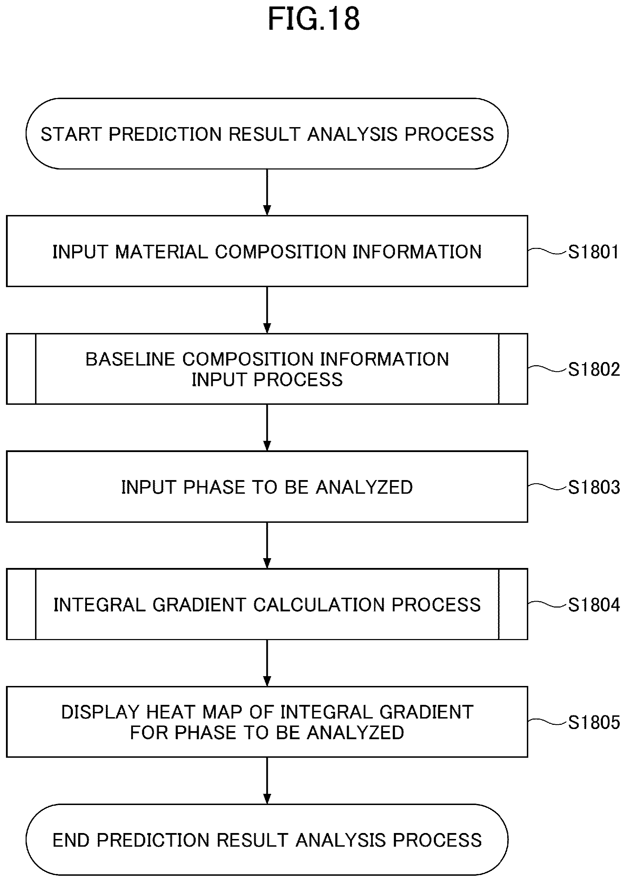

Next, a flow of a prediction result analysis process by the prediction result analysis device 130 will be described. FIG. 18 is a flow chart illustrating the flow of the prediction result analysis process.

In step S1801, the material composition input unit 131 receives an input of the material composition information of the material to be predicted.

In step S1802, the baseline composition input unit 132 receives an input of the baseline composition information. A detailed flow chart of a baseline composition information input process will be described later.

In step S1803, the prediction unit 134 receives an input of the phase to be analyzed.

In step S1804, the intermediate composition generation unit 133 generates the intermediate composition information, based on the material composition information of the material to be predicted and the baseline composition information. The prediction unit 134 predicts the phase ratio based on the generated intermediate composition information, and derives the partial derivative for each temperature and each component with respect to the phase to be analyzed. The integral gradient calculation unit 135 calculates the integral gradient of each temperature and each component, by integrating the derived partial derivative for each temperature and each component. A detailed flow chart of an integral gradient calculation process by the intermediate composition generation unit 133, the prediction unit 134, and the integral gradient calculation unit 135 will be described later.

In step S1805, the display unit 136 displays a heat map of the integral gradient, with respect to the phase to be analyzed.

As described above, according to the prediction result analysis device 130, it is possible to understand the degree of impact of the content of each additive element on the phase ratio, by merely inputting a plurality of baseline composition information and the phase to be analyzed.

That is, it is not necessary to perform an operation of predicting the phase ratio within the specific temperature range while successively increasing and decreasing the content of all of the additive elements, and it is easy to understand the degree of impact of the content of each additive element on the phase ratio.

### <Detailed Flow of Baseline Composition Information Input Process>

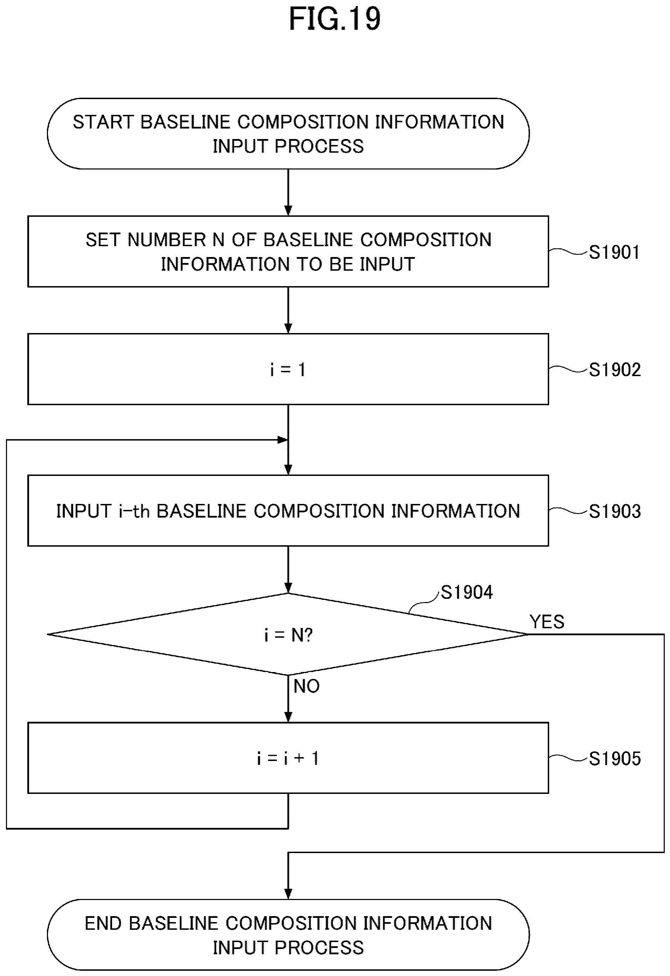

Next, a detailed flow of the baseline composition information input process (step S1802) will be described. FIG. 19 is a flow chart illustrating a flow of the baseline composition information input process.

In step S1901, the baseline composition input unit 132 receives a setting of the number (= N) of baseline composition information from the designer of the material design.

In step S1902, the baseline composition input unit 132 sets "1" to a counter i for counting the number of baseline composition information.

In step S1903, the baseline composition input unit 132 receives an input of an i-th baseline composition information from the designer of the material design.

In step S1904, the baseline composition input unit 132 determines whether or not the counter i reached a count N. When it is determined in step S1904 that the counter i has not reached the count N (NO in step S1904), the process proceeds to step S1905.

In step S1905, the baseline composition input unit 132 increments the counter i, and the process thereafter returns to step S1903.

On the other hand, when it is determined in step S1905 that the counter i reached the count N (YES in step S1905), the baseline composition information input process is ended.

### <Detailed Flow of Integral Gradient Calculation Process>

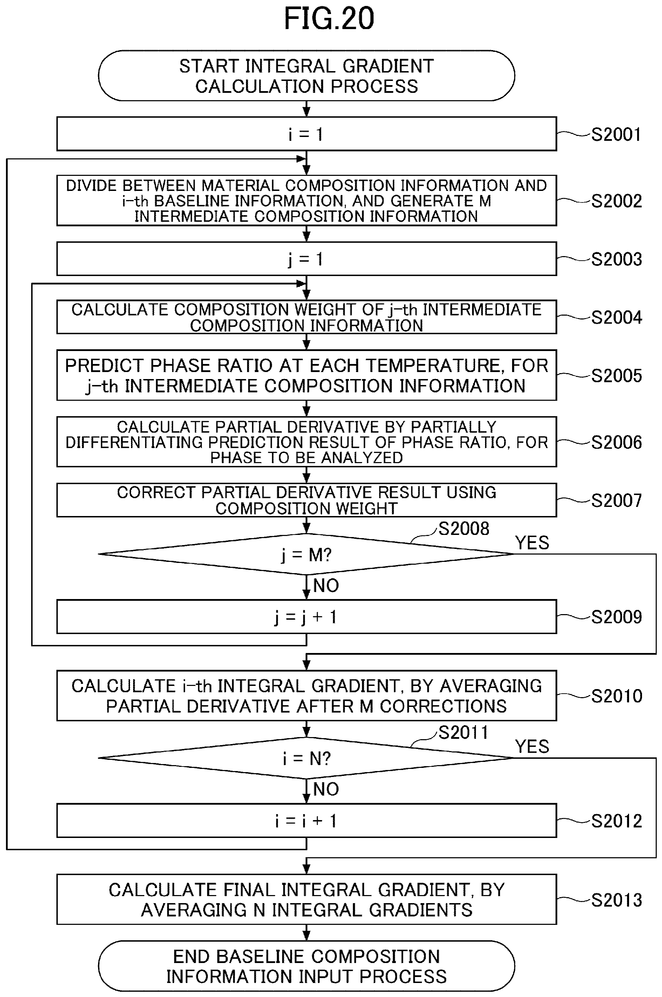

Next, a detailed flow of the integral gradient calculation process (step S1804) will be described. FIG. 20 is a flow chart illustrating the flow of the integral gradient calculation process.

In step S2001, the intermediate composition generation unit 133 sets "1" to the counter i that counts the number of baseline composition information.

In step S2002, the intermediate composition generation unit 133 generates M intermediate composition information by dividing between the material composition information of the material to be predicted and the i-th baseline composition information, by the number of steps = M.

In step S2003, the intermediate composition generation unit 133 sets "1" to a counter j for counting a number of generated intermediate composition information.

In step S2004, the intermediate composition generation unit 133 calculates a composition weight of a j-th intermediate composition information. The intermediate composition generation unit 133 calculates the composition weight, based on a difference between the material composition information of the material to be predicted and the j-th intermediate composition information.

In step S2005, the prediction unit 134 predicts the phase ratio at each temperature within the specific temperature range, by inputting the j-th intermediate composition information to the trained prediction model.

In step S2006, the prediction unit 134 derives the partial derivative for each temperature and each constituent, by performing a partial differentiation on the phase to be analyzed, using the j-th intermediate composition information.

In step S2007, the integral gradient calculation unit 135 corrects the partial derivative derived for each temperature and each component, using the composition weight, respectively.

In step S2008, the intermediate composition generation unit 133 determines whether or not the counter j for counting the number of intermediate composition information reached a count M. When it is determined in step S2008 that the counter j has not reached the count M (NO in step S2008), the process proceeds to step S2009.

In step S2009, the intermediate composition generation unit 133 increments the counter j, and the process thereafter returns to step S2004.

On the other hand, when it is determined in step S2008 that the counter j reached the count M (YES in step S2008), the process proceeds to step S2010.

In step S2010, the integral gradient calculation unit 135 integrates the M corrected partial derivatives derived for each temperature and each component, for each temperature and each component, and thereafter divides the integrated corrected partial derivatives by M, in order to average the corrected integrated corrected partial derivatives. Thus, the integral gradient calculation unit 135 calculates the integral gradient of each temperature and each component corresponding to the i-th baseline information.

In step S2011, the intermediate composition generation unit 133 determines whether or not the counter i for counting the number of baseline composition information reached a count N. When it is determined in step S2011 that the counter i has not reached the count N (NO in step S2011), the process proceeds to step S2012.

In step S2012, the intermediate composition generation unit 133 increments the counter i, and the process thereafter returns to step S2002.

On the other hand, when it is determined in step S2011 that the counter i reached the count N (YES in step S2011), the process proceeds to step S2013.

In step S2013, the N integral gradients for each temperature and each component are integrated for each temperature and each constituent, and the integrated integral gradients are averaged by dividing by N. Thus, the integral gradient calculation unit 135 can calculate the final integral gradient, that is, the integral gradient after the averaging, with respect to the material composition information of the material to be predicted.

### <Specific Example of Heat Map>

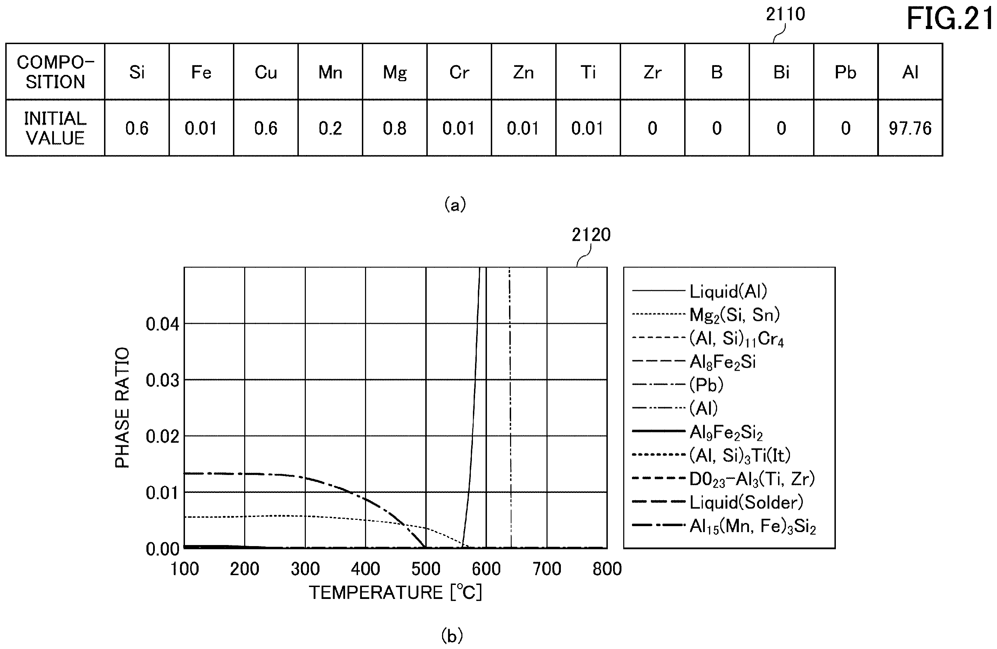

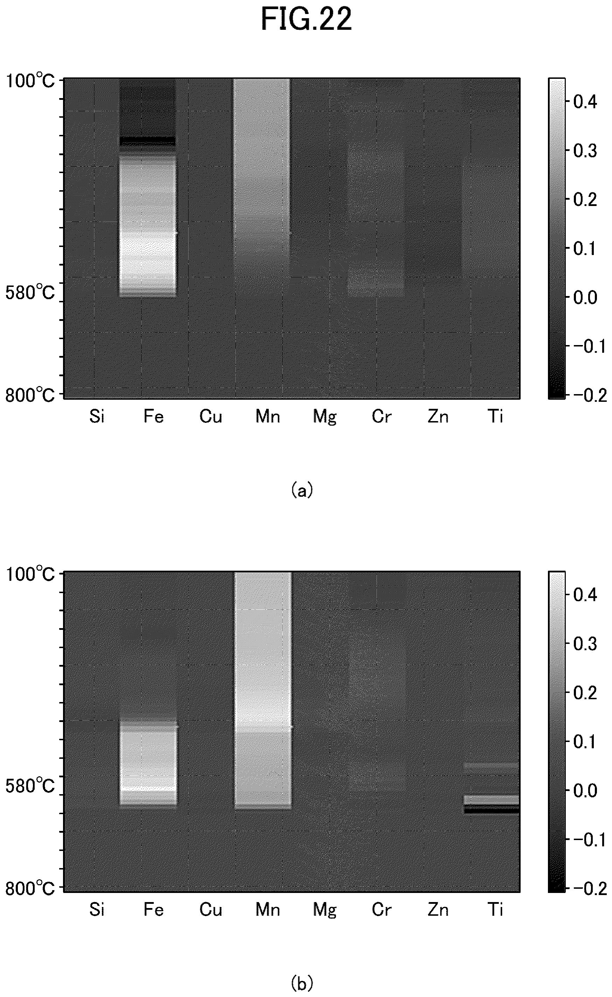

Next, a specific example of the heat map of the integral gradient calculated by the prediction result analysis process (FIG. 18) by the prediction result analysis device 130 will be described with reference to FIG. 21 and FIG. 22. FIG. 21 is a diagram illustrating an example of the material composition of the material to be predicted and the predicted phase ratio used in the prediction result analysis process. FIG. 22 is a diagram illustrating an example of a heat map displayed in a display mode corresponding to the integral gradient.

As illustrated in FIG. 21, when the material composition information of the material to be predicted is the material composition information indicated by a reference numeral 2110, the prediction unit 122 predicts a reference numeral 2120 as the phase ratio at each of the temperatures from 100°C to 800°C. In contrast, when the designer of the material design sets Al₁₅ (Mn, Fe)₃Si₂ as the phase to be analyzed, and the prediction result analysis device 130 calculates the heat map of the integral gradient, the heat maps illustrated in (a) and (b) of FIG. 22 are obtained.

Each of (a) and (b) of FIG. 22 illustrates the heat map for the case where "1" is set as the number (= N) of baseline composition information. However, it is assumed that mutually different baseline composition information is input between (a) and (b) of FIG. 22.

In a case where the designer of the material design increases or decreases each additive element (Si, Fe, Cu, Mn, Mg, Cr, Zn, and Ti) included in the material to be predicted by referring to FIG. 22, the designer of the material design can recognize:
- the degree of impact of each added element with respect to the Al₁₅(Mn, Fe)₃Si₂ which is the phase to be analyzed; and
- the temperature or a vicinity of the temperature at which the impact occurs.

As described above, according to the prediction result analysis device 130, the heat map is displayed in the display mode corresponding to the integral gradient calculated for each temperature and each component with respect to the phase to be analyzed, and thus, the designer can easily understand the degree of impact of the content of each additive element on the phase ratio.

That is, compared to the case where the phase ratios before and after the modification of the content are compared, the designer can easily understand the degree of impact of the content of each additive element on the phase ratio.

In the examples in (a) and (b) of FIG. 22, the number of baseline composition information is "1", but the number of baseline composition information may further be increased in order to calculate the degree of impact of each additive element on the phase to be analyzed with a higher accuracy.

In addition, in the examples in (a) and (b) of FIG. 22, the number of steps = M is not mentioned, but by further increasing the number of steps = M, the degree of impact of each additive element on the phase to be analyzed can be calculated with a higher accuracy.

### <Conclusion>

As is clear from the above description, the prediction result analysis device 130 according to the first embodiment includes the following features:
- The storage unit configured to store a trained prediction model that is trained using training data in which the material composition of the material to be trained, and the phase ratio of the material to be trained at each temperature within the specific temperature range, are associated with each other.
- The material composition of a plurality of intermediate materials is generated, by dividing between the material composition of the material to be predicted and the material composition of the baseline material.
- The phase ratio of the plurality of intermediate materials at each temperature within the specific temperature range is predicted, by inputting the material composition of the plurality of intermediate material to the trained prediction model.
- When the phase ratio is predicted, a partial derivative is calculated by partially differentiating a value predicted with respect to the phase to be analyzed at each temperature using the material composition of the plurality of intermediate materials.
- The calculated partial differential value is integrated for each component included in the material composition of the plurality of intermediate materials at each temperature, thereby calculating the integral gradient of each component at each temperature.
- In a heat map in which each component is arranged on a first axis and each temperature is arranged on a second axis, each area specified based on each component and each temperature is displayed in a display mode corresponding to the integral gradient.

Thus, according to the first embodiment, it is possible to easily understand the degree of impact on the phase ratio caused by the modification of the material composition.

### [Second Embodiment]

In the first embodiment, the color scheme of the heat map is a color scheme indicated in (a) and (b) of FIG. 22, but a color scheme other than the color scheme indicated in (a) and (b) of FIG. 22 may be employed. However, the heat map may be represented in a display mode other than the color scheme according to the integral gradient.

The first embodiment describes a case where the integrated gradient is represented using the heat map, so that the designer can easily understand the content of which of the additive elements can be increased or decreased to impact the phase ratio of which temperature range. However, the method of expressing the integrated gradient is not limited to using the heat map. For example, in a case where it is desirable to understand which temperature within the specific temperature range has a large degree of impact, a graph representing the transition of the degree of impact due to the difference in temperature may be displayed in place of the heat map.

Although the first embodiment describes the alloy composition indicating the content of the additive element, such as another metal element or a non-metal element, with respect to the metal element included in the alloy as a specific example of the material composition, the material composition is not limited to the alloy composition. For example, a chemical composition may indicate the content of each chemical component included in a substance other than the alloy.

In the first embodiment, the Seq2Seq with Attention mechanism or the Transformer is implemented as the trained prediction model. However, the trained prediction model is not limited to such, and other architectures may be implemented as long as the architecture can process the time series data, that are the data at specific time intervals. For example, a recurrent neural network (RNN), a bidirectional RNN, a Seq2Seq, or the like may be implemented as the trained prediction model. Alternatively, a gated recurrent unit (GRU), a long short term memory (LSTM), or the like may be implemented as the trained prediction model.

Although the training device 110, the prediction device 120, and the prediction result analysis device 130 are configured as separate devices in the first embodiment, the training device 110, the prediction device 120, and the prediction result analysis device 130 may be configured as a single device. Alternatively, two of the training device 110, the prediction device 120, and the prediction result analysis device 130 may be configured as a single device.

The present invention is not limited to the configurations or the like described in the embodiments, and combinations with other elements or the like are possible. These modifications can be made without departing from the scope of the present invention, and can be appropriately determined according to the form of application.

This application is based upon and claims priority to Japanese Patent Application No. 2023-038608 filed on March 13, 2023, the entire contents of which are incorporated herein by reference.

### DESCRIPTION OF REFERENCE NUMERALS

100: prediction system
110: training device
111: training data generation unit
112: training unit
120: prediction device
121: material composition input unit
122: prediction unit
123: display unit
130: prediction result analysis device
131: material composition input unit
132: baseline composition input unit
133: intermediate composition input unit
134: prediction unit
135: integral gradient calculation unit
136: display unit
300: training data
401: element information input unit
402: combination determination unit
403: simulation unit
404: storage control unit
501: encoder unit
502: decoder unit
503: loss function calculation unit
801: phase ratio error calculation unit at
generation/loss temperature
802: phase ratio error calculation unit
803: phase ratio error (logarithmic value) calculation unit
804: phase ratio error (difference value) calculation unit
805: cross entropy error calculation unit
806: weighted adder unit
1001: trained encoder unit
1002: trained decoder unit
1501: phase specification unit
1502: sensitivity characteristic calculation unit

## Claims

1. A prediction result analysis device comprising:
a storage unit configured to store a trained model that is trained using training data in which a material composition of a material to be trained, and a phase ratio of the material to be trained at each temperature within a specific temperature range, are associated with each other, the trained model being configured to predict a phase ratio at an (i+1)-th temperature using phase ratios predicted by the trained model for temperatures to an i-th temperature (i is an integer greater than or equal to 1) within the specific temperature range;
an intermediate composition generation unit configured to generate a material composition of a plurality of intermediate materials, by dividing between the material composition of the material to be predicted and a material composition of a baseline material;
a prediction unit configured to calculate a partial derivative by partially differentiating a value predicted for a phase specified in advance at each temperature, when predicting a phase ratio of the plurality of intermediate materials at each temperature within the specific temperature range, by inputting the material composition of the plurality of intermediate materials to the trained prediction model;
an integral gradient calculation unit configured to calculate an integral gradient of each temperature and each component, by integrating the calculated partial derivative for each component included in the material composition of the plurality of intermediate materials at each temperature; and
a display unit configured to display a heat map in which each component is arranged on a first axis and each temperature is arranged on a second axis, and each area specified based on each component and each temperature is displayed in a display mode corresponding to the integral gradient.

2. The prediction result analysis device as claimed in claim 1, wherein the integral gradient calculation unit calculates the integral gradient of each temperature and each component, by integrating the calculated partial derivative of each temperature for each component included in the material composition of the plurality of intermediate materials using a composition weight calculated in advance for each intermediate material of the plurality of intermediate materials, and averaging the integrated partial derivative.

3. The prediction result analysis device as claimed in claim 2, wherein the integral gradient calculation unit calculates the composition weight, based on a difference between each material composition of the plurality of intermediate materials, and the material composition of the material to be predicted.

4. The prediction result analysis device as claimed in claim 3, wherein the integral gradient calculation unit calculates the composition weight, based on a difference between each material composition of the plurality of intermediate materials, and the material composition of the material to be predicted.

5. The prediction result analysis device as claimed in any one of claims 1 to 4, wherein:
the intermediate composition generation unit generates the material composition of the plurality of intermediate materials, for each material composition of a plurality of baseline materials,
the integral gradient calculation unit calculates the integral gradient of each temperature and each component, for each material composition of the plurality of baseline materials, and
the display unit displays the heat map in a display mode according to an averaged integral gradient acquired by averaging a plurality of integral gradients of each temperature and each component.

6. The prediction result analysis device as claimed in any one of claims 1 to 5, wherein an architecture capable of processing time series data which are data at specific time intervals is applied to the trained model, and the trained model predicts a phase ratio for each specific temperature interval based on the material composition of the material to be predicted.

7. The prediction result analysis device as claimed in claim 6, wherein the trained model is any one of an RNN, a bidirectional RNN, a Seq2Seq, a Seq2Seq with Attention mechanism, a GRU, an LSTM, and a Transformer.

8. The prediction result analysis device as claimed in claim 7, wherein the trained model includes:
an encoder unit configured to output a feature, in response to an input of the material composition of the material to be predicted, and
a decoder unit configured to predict the phase ratio at the (i+1)-th temperature, in response to an input of the output feature and predicted phase ratios for the temperatures to the i-th temperature.

9. The prediction result analysis device as claimed in any one of claims 1 to 8, wherein the phase ratio is a phase ratio in a thermodynamic equilibrium state.

10. A prediction result analysis method to be implemented in a computer for a trained model that is trained using training data in which a material composition of a material to be trained, and a phase ratio of the material to be trained at each temperature within a specific temperature range, are associated with each other, the trained model being configured to predict a phase ratio at an (i+1)-th temperature using phase ratios predicted by the trained model for temperatures to an i-th temperature (i is an integer greater than or equal to 1) within the specific temperature range, the prediction result analysis method comprising the steps of:
generating, by the computer, a material composition of a plurality of intermediate materials, by dividing between the material composition of the material to be predicted and a material composition of a baseline material;
calculating, by the computer, a partial derivative by partially differentiating a value predicted for a phase specified in advance at each temperature, when predicting a phase ratio of the plurality of intermediate materials at each temperature within the specific temperature range, by inputting the material composition of the plurality of intermediate materials to the trained prediction model;
calculating, by the computer, an integral gradient of each temperature and each component, by integrating the calculated partial derivative for each component included in the material composition of the plurality of intermediate materials at each temperature; and
displaying, by the computer, a heat map in which each component is arranged on a first axis and each temperature is arranged on a second axis, and each area specified based on each component and each temperature is displayed in a display mode corresponding to the integral gradient.

11. A prediction program for causing a computer to implement a trained model that is trained using training data in which a material composition of a material to be trained, and a phase ratio of the material to be trained at each temperature within a specific temperature range, are associated with each other, the trained model being configured to predict a phase ratio at an (i+1)-th temperature using phase ratios predicted by the trained model for temperatures to an i-th temperature (i is an integer greater than or equal to 1) within the specific temperature range, the prediction program causing the computer to perform the steps of:
generating a material composition of a plurality of intermediate materials, by dividing between the material composition of the material to be predicted and a material composition of a baseline material;
calculating a partial derivative by partially differentiating a value predicted for a phase specified in advance at each temperature, when predicting a phase ratio of the plurality of intermediate materials at each temperature within the specific temperature range, by inputting the material composition of the plurality of intermediate materials to the trained prediction model;
calculating an integral gradient of each temperature and each component, by integrating the calculated partial derivative for each component included in the material composition of the plurality of intermediate materials at each temperature; and
displaying a heat map in which each component is arranged on a first axis and each temperature is arranged on a second axis, and each area specified based on each component and each temperature is displayed in a display mode corresponding to the integral gradient.
